# EUROPEAN PATENT APPLICATION

(11) **EP 4 144 759 A1**
(43) Date of publication of application: **08.03.2023**
(21) Application number: 21796383.4
(22) Date of filing: 25.04.2021
(51) Int. Cl.: C07K 16/28, C07K 19/00, A61K 39/395, A61P 35/00, A61P 31/00, A61P 37/00

(54) **BISPECIFIC ANTIBODY TARGETING HUMAN CLAUDIN AND HUMAN PDL1 PROTEINS, AND APPLICATION THEREOF**

(30) Priority: 27.04.2020 CN 202010344676
(71) Applicant: Qure Biotechnology (Shanghai) Co., Ltd., Shanghai 200120 (CN)
(72) Inventor: QU, Xiangdong, Shanghai 200120 (CN); PAN, Qin, Shanghai 200120 (CN); JIN, Houcong, Shanghai 200120 (CN); ZHENG, Han, Shanghai 200120 (CN); DU, Yejie, Shanghai 200120 (CN)
(74) Representative: Dehns
(86) International application number: PCT/CN2021/089729
(87) International publication number: WO 2021/218874

(57) **Abstract**

A bispecific antibody targeting human claudin and human PDL1 proteins, containing an anti-human claudin18.2 antibody moiety and an anti-PD-L1 antibody moiety. The bispecific antibody can not only bind to human claudin18.2 protein, but also block binding of PD-1/PD-L1, and can not only activate NK cells and kill tumor cells in the innate immunity, but also promote the killing effect of killer T lymphocytes on tumors in the acquired immunity. The bispecific antibody has better anti-tumor efficacy than an anti-claudin18.2 antibody alone.

## Description

### Technical Field

The present disclosure relates to a bispecific antibody targeting human claudin and human PDL1 proteins and application thereof, and belongs to the field of biomedicine.

### Background

A bispecific antibody (BsAb), also known as a bifunctional antibody, can recognize and bind to two different antigens and epitopes simultaneously and block two different signalling pathways to play its role. As compared with a conventional antibody, the BsAb has one more specific antigen-binding site, which may lead to advantages in the following aspects of treatments.

Mediation of killing of tumors by immune cells: one important action mechanism of the bispecific antibody is to mediate the killing conducted by the immune cells; a bispecific antibody has two antigen-binding arms, one of which binds to a target antigen and the other binds to a labelled antigen on effector cells, and the latter can activate the effector cells for targeted killing of tumor cells.

Double-target signal blocking, which plays a unique or overlapping functions and effectively prevents drug resistance: simultaneously binding to double targets and blocking dual signalling pathways is another important mechanism of action for bispecific antibodies. Receptor tyrosine kinases (receptor tyrosine kinases, RTKs), such as the Her family, are the largest class of enzyme-linked receptors, which play important regulatory roles during cell proliferation. The RTKs are abnormally highly expressed on the surface of tumor cells, which leads to the malignant proliferation of tumor cells, therefore they are important targets for tumor therapy. Single-target monoclonal antibodies against RTKs have been widely used in tumor therapy; however, tumor cells can undergo immune escape through switching signalling pathways or activating intracellular signals by the homodimers of the members themselves or the heterodimers of different members of the HER family. Therefore, the use of drugs of a bispecific antibody to simultaneously block two or more RTKs or their ligands can reduce the escape of tumor cells and improve therapeutic effects.

Stronger specificity and targeting and reduced off-target toxicity: by means of utilizing the characteristics that the two antigen-binding arms of a bispecific antibody can bind different antigens, the two antigen-binding arms respectively bind 2 kinds of antigens on the surface of cancer cells, and the binding specificity and targeting of the antibody to cancer cells can be effectively enhanced and the side effects such as off-target can be reduced;

Effective reduction of cost for treatment: take BiTE as an example, it has stronger competitiveness in tissue permeability, efficiency of killing tumor cells, off-target rate, clinical indications and other indicators as compared with traditional antibodies, and has significant clinical advantages. Especially with regard to the dosage used, since the therapeutic effect thereof can reach 100-1000 times of that of conventional antibody and the lowest dosage used thereof will be 1/2000 of the original dosage, the cost of drug treatment is thus significantly reduced. As compared with combination therapy, the cost of bispecific antibody is much lower than that of the combination therapy of two single drugs.

PD-1(CD279) was first reported in 1992; the coding gene *PDCD1* for human PD-1 is located at 2q37.3, with a total length of 2097bp, consisting of 6 exons; the translation product thereof is a PD-1 precursor protein consisting of 288 amino acids, and a mature protein is obtained by cleaving the signal peptide consisting of the first 20 amino acids. PD-1 includes an IgV domain of extracellular immunoglobulin variable region, a hydrophobic transmembrane domain and an intracellular domain; the ITIM motif at the N-terminal of the intracellular tail domain contains 2 phosphorylation sites, and a ITSM motif is located on the C-terminal. PD-1 is a membrane protein, belonging to CD28 immunoglobulin superfamily; it is mainly expressed on the surface of activated T cells; in addition, it is also expressed in low abundance in CD4⁻CD8⁻T cells, activated NK cells and monocytes in thymus. PD-1 has 2 ligands, namely PD-L1 (CD274, B7-H1) and PD-L2 (CD273, B7-DC) of B7 protein family; and 40% of the amino acid sequences of PD-L1 and PD-L2 are identical. The difference between the two ligands mainly lies in the different expression patterns; the constitutive low expression of PD-L1 is found in APCs, non-hematopoietic cells (such as vascular endothelial cells and islet cells) and immune privilege sites (such as placenta, testis and eyes); and inflammatory cytokines such as type I and type II interferon, TNF-α and VEGF can induce the expression of PD-L1. However, PD-L2 is only expressed in activated macrophages and dendritic cells. The ITSM motif of PD-1 undergoes tyrosine phosphorylation after the binding of PD-1 and PD-L1 in activated T cells, which in return leads to the dephosphorylation of downstream protein kinases Syk and PI3K, inhibits the activation of downstream pathways such as AKT and ERK, and finally inhibits the transcription and translation of genes and cytokines required for T cell activation, which plays a negative role in regulating T cell activity.

In tumor cells, tumor cells and tumor microenvironment negatively regulate T cell activity and inhibit immune response by up-regulating the expression of PD-L1 and binding with PD-1 on the surface of tumor-specific CD8⁺T cells. Tumor cells can up-regulate the expression of PD-L1 through the following 4 ways: 1. amplification of the gene encoding PD-L1 (9p24.1); 2. activation ofEGFR, MAPK and PI3K-Akt signaling pathways, and up-regulation of expression of PD-L1 at transcription level via HIF-1 transcription factor and the like; 3. induction by EB virus (EB virus positive gastric cancer and nasopharyngeal carcinoma showed high expression of PD-L1); 4. regulation of epigenetics. In tumor microenvironment, the stimulation of inflammatory factors such as interferon-γ can also induce the expression of PD-L1 and PD-L2. Inflammatory factors can induce other cells including macrophages, dendritic cells and stromal cells in tumor microenvironment to express PD-L1 and PD-L2, while tumor infiltrating T cells capable of recognizing tumor antigens can secrete interferon-γ, thus inducing the up-regulation of PD-L1 expression, in which this process is called "adaptive immune resistance", and tumor cells can protect themselves through this mechanism. There is more and more evidence that tumors escape from immune by utilizing PD-1-dependent immunosuppression. It has been found that PD-L1 and PD-L2 were highly expressed in various solid tumors and hematological malignancies. In addition, there is a strong correlation between the expression of PD-Ls and the poor prognosis of tumor cells, which has been proved to include esophageal cancer, gastric cancer, renal cancer, ovarian cancer, bladder cancer, pancreatic cancer and melanoma.

In the past few decades, the morbidity and mortality of gastric cancer in the United States and many developed countries have decreased significantly. However, a cancer originating in stomach (GC), esophagus or gastroesophageal junction (GEJ) is still a major global health problem, especially in low-income and middle-income countries. The global incidence of gastric cancer shows wide geographical difference, and the difference between high incidence and low incidence areas is 15 to 20 times. Globally, it was estimated that 1.03 million cases of gastric cancer caused more than 780,000 deaths in 2018, causing gastric cancer the fifth most commonly diagnosed cancer and the third leading cause of cancer-related death in the world; however, gastric cancer is one of the rare cancers in Western Europe, Australia and North America. In 2019, it was estimated that 27,510 people would be diagnosed and 11,140 people would die from this disease in the United States; and it was the 15th most commonly diagnosed cancer and the 15th leading cause of cancer-related death in the United States. The highest incidence of gastric cancer occurs in East Asia, South America, Central America and Eastern Europe, among which the incidence of gastric cancer is particularly high in three East Asian countries (China, Japan and South Korea). In China, gastric cancer is the most common cancer among men and the main cause of cancer-related mortality. In recent years, claudin-18A2 (Claudin18.2) which has been regarded as a tumor-specific antigen of digestive tract tumors especially gastric cancer has attracted more and more attention.

Tight junction (TJ) plays a key role in cell-to-cell material flow, it also maintains cell polarity by blocking the radial diffusion of membrane proteins and membrane lipids, and additionally participates in recruiting signal molecules that regulate cell proliferation, differentiation and movement. Tight junctions are formed by claudin (Claudin, CLDN), and the family of claudin consists of more than 20 protein molecules, each member of which contains a tetra-transmembrane domain and a similar amino acid sequence, but the tissue distribution thereof has some specificity. CLDN plays a key role in regulating the selective permeation of cell bypass; CLDN2 and CLDN15 participate in the formation of cation channels and cation pores, while CLDN4/7/10 participates in the formation of anion channels and pores. The differential expression of CLDN proteins is thought to be associated with many cancers. Down-regulation of CLDN1 and CLDN7 occurred in invasive breast cancer, prostate cancer and esophageal cancer, while it was found that CLDN3/4 up-regulation in different degrees occurred in various cancers such as cervical cancer, colon cancer, esophageal cancer and gastric cancer. Sahin *et al.* found that in normal tissues, the isoform 2 subtype of CLDN18 (Claudin18.2) was only expressed in differentiated epidermal cells of gastric mucosa, but did not express in gastric stem cells; however, abnormally high expression was found in primary gastric cancer and metastatic foci thereof. Overexpression of Claudin18.2 has also been reported in pancreatic cancer, esophageal cancer and lung cancer. Since Claudin18.2 is located on the surface of cell membrane, its biological function and characteristics determine that it is an ideal therapeutic target; and in recent years, monoclonal antibodies against this target have also come into being.

### Content of the present disclosure

An objective of the present disclosure is to provide a bispecific antibody targeting human claudin and human PDL1 proteins and use thereof.

The present disclosure employs the following technical solutions:
A bispecific antibody targeting human claudin18.2 and human PDL1 proteins including:
an anti-human claudin18.2 antibody moiety and an anti-PD-L1 antibody moiety.

Furthermore, the sequence of the bispecific antibody of the present disclosure targeting human claudin18.2 and human PDL1 proteins is as shown in: SEQ ID NO: 59, SEQ ID NO: 60, SEQ ID NO: 61, SEQ ID NO: 62, SEQ ID NO: 63, SEQ ID NO: 64, SEQ ID NO: 65 or SEQ ID NO: 66.

Furthermore, a bispecific antibody of the present disclosure targeting human claudin18.2 and human PDL1 proteins, in which the anti-human claudin18.2 antibody moiety binds to the extracellular region of human claudin18.2 protein, the sequence of the anti-human claudin18.2 antibody moiety is as shown in: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33 or SEQ ID NO: 34.

Furthermore, a bispecific antibody of the present disclosure targeting human claudin18.2 and human PDL1 proteins, in which the sequence of the anti-PD-L1 antibody is as shown in SEQ ID NO: 49, SEQ ID NO: 50, SEQ ID NO: 51, SEQ ID NO: 52, SEQ ID NO: 53 or SEQ ID NO: 54.

Disclosed is use of any one of the above-described bispecific antibodies in preparing of a medicament for treating cancer, infection or immunoregulatory diseases.

Disclosed is use of any one of the above-described bispecific antibodies in preparing of a medicament for inhibiting tumor growth.

Furthermore, the cancer or tumor is selected from the following groups or sites: colorectum, breast, ovary, pancreas, stomach, esophagus, prostate, kidney, cervix, bone marrow cancer, lymphoma, leukemia, thyroid, endometrium, uterus, bladder, neuroendocrine, head and neck, liver, nasopharynx, testis, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

Specifically, in a first aspect of the present disclosure, a bispecific antibody targeting human claudin18.2 and human PD-L1 proteins is provided, which the bispecific antibody including:
an anti-human claudin18.2 antibody moiety and an anti-PD-L1 antibody moiety.

In another preferred embodiment, the bispecific antibody has both the activity of binding to a human claudin18.2 and the activity of binding to a human PD-L1 protein.

In another preferred embodiment, the complementarity determining region CDR of the anti-human claudin18.2 antibody includes:
HCDR1 as shown in SEQ ID NO: 93, 75, 79, 83 or 87,
HCDR2 as shown in SEQ ID NO: 94, 76, 80, 84 or 88, and
HCDR3 as shown in SEQ ID NO: 95, 77, 81, 85 or 89; and
LCDR1 as shown in SEQ ID NO: 90 or 72,
LCDR2 as shown in SEQ ID NO: 91 or 73, and
LCDR3 as shown in SEQ ID NO: 92, 74, 78, 82 or 86.

In another preferred embodiment, 3 heavy chain CDRs and 3 light chain CDRs of anti-human claudin18.2 antibody are selected from the following groups:
(Z1) HCDR1, HCDR2 and HCDR3 as shown in SEQ ID NO: 93, 94, and 95; and LCDR1, LCDR2 and LCDR3 as shown in SEQ ID NO: 90, 91, and 92;
(Z2) HCDR1, HCDR2 and HCDR3 as shown in SEQ ID NO: 75, 76 and 77; and LCDR1, LCDR2 and LCDR3 as shown in SEQ ID NO: 72, 73 and 74;
(Z3) HCDR1, HCDR2 and HCDR3 as shown in SEQ ID NO: 79, 80 and 81; and LCDR1, LCDR2 and LCDR3 as shown in SEQ ID NO: 72, 73 and 78;
(Z4) HCDR1, HCDR2 and HCDR3 as shown in SEQ ID NO: 83, 84 and 85; and LCDR1, LCDR2 and LCDR3 as shown in SEQ ID NO: 72, 73, and 82;
(Z5) HCDR1, HCDR2 and HCDR3 as shown in SEQ ID NO: 87, 88, and 89; and LCDR1, LCDR2 and LCDR3 as shown in SEQ ID NO: 72, 73 and 86.

In another preferred embodiment, the CDRs of the anti-human claudin18.2 antibody includes the HCDR1 as shown in SEQ ID NO: 93, the HCDR2 as shown in SEQ ID NO: 94 and the HCDR3 as shown in SEQ ID NO: 95, and the LCDR1 as shown in SEQ ID NO: 90, the LCDR2 as shown in SEQ ID NO: 91 and the LCDR3 as shown in SEQ ID NO: 92.

In another preferred embodiment, the anti-PD-L1 antibody is a single-domain antibody.

In another preferred embodiment, 3 complementarity determining regions (CDRs) of the single-domain antibody include the HCDR1 as shown in SEQ ID NO: 69, the HCDR2 as shown in SEQ ID NO: 70 or 96 and the HCDR3 as shown in SEQ ID NO: 71.

In another preferred embodiment, 3 complementarity determining regions (CDRs) of the single-domain antibody include the HCDR1 as shown in SEQ ID NO: 69, the HCDR2 as shown in SEQ ID NO: 70 and the HCDR3 as shown in SEQ ID NO: 71.

In another preferred embodiment, 3 complementarity determining regions (CDRs) of the single-domain antibody include the HCDR1 as shown in SEQ ID NO: 69, the HCDR2 shown in SEQ ID NO: 96 and the HCDR3 shown in SEQ ID NO: 71.

In another preferred embodiment, the bispecific antibody is a dimer composed of two monomers, and the monomers have the structure shown in formula I from the N terminus to the C terminus: 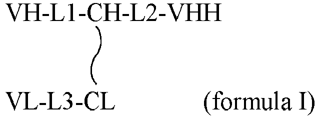 wherein,
L1, L2 and L3 are each independently a bond or a linker element;
VH represents the heavy chain variable region of the anti-human claudin18.2 antibody;
VL represents the light chain variable region of the anti-human claudin18.2 antibody;
CH represents the heavy chain constant region of the anti-human claudin18.2 antibody;
CL represents the light chain constant region of the anti-human claudin18.2 antibody;
VHH represents anti-PD-L1 single-domain antibody;
"-"represents peptide bond;
" ∼ " represents a disulfide bond or covalent bond. In another preferred embodiment, L1 and L3 are each a bond (such as a peptide bond). In another preferred embodiment, the heavy chain variable region (VH) of the anti-human claudin18.2 antibody includes the HCDR1 as shown in SEQ ID NO: 93, 75, 79, 83 or 87, the HCDR2 as shown in SEQ ID NO: 94, 76, 80, 84 or 88 and the HCDR3 as shown in SEQ ID NO: 95, 77, 81, 85 or 89.

In another preferred embodiment, the heavy chain variable region (VH) of the anti-human claudin18.2 antibody further includes a humanized FR region.

In another preferred embodiment, the amino acid sequence of the heavy chain variable region (VH) of the anti-human claudin18.2 antibody is as shown in SEQ ID NO: 31, SEQ ID NO: 4, SEQ ID NO: 5, SEQ ID NO: 6, SEQ ID NO: 11, SEQ ID NO: 12, SEQ ID NO: 13, SEQ ID NO: 17, SEQ ID NO: 18, SEQ ID NO: 19, SEQ ID NO: 24, SEQ ID NO: 25, SEQ ID NO: 26, SEQ ID NO: 27, SEQ ID NO: 32, SEQ ID NO: 33 or SEQ ID NO: 34.

In another preferred embodiment, the light chain variable region (VL) of the anti-human claudin18.2 antibody includes the LCDR1 as shown in SEQ ID NO: 90 or 72, the LCDR2 as shown in SEQ ID NO: 91 or 73 and the LCDR3 as shown in SEQ ID NO: 92, 74, 78, 82 or 86.

In another preferred embodiment, the light chain variable region (VL) of the anti-human claudin18.2 antibody further includes a humanized FR region.

In another preferred embodiment, the amino acid sequence of the light chain variable region (VL) of the anti-human claudin18.2 antibody is as shown in SEQ ID NO: 29, SEQ ID NO: 1, SEQ ID NO: 2, SEQ ID NO: 3, SEQ ID NO: 7, SEQ ID NO: 8, SEQ ID NO: 9, SEQ ID NO: 10, SEQ ID NO: 14, SEQ ID NO: 15, SEQ ID NO: 16, SEQ ID NO: 20, SEQ ID NO: 21, SEQ ID NO: 22, SEQ ID NO: 23, SEQ ID NO: 28 or SEQ ID NO: 30.

In another preferred embodiment, the heavy chain constant region (CH) of the anti-human claudin18.2 antibody is human-derived or mouse-derived.

In another preferred embodiment, the light chain constant region (CL) of the anti-human claudin18.2 antibody is human-derived or mouse-derived.

In another preferred embodiment, the anti-PD-L1 single-domain antibody (VHH) includes the HCDR1 as shown in SEQ ID NO: 69, the HCDR2 as shown in SEQ ID NO: 70 and the HCDR3 as shown in SEQ ID NO: 71.

In another preferred embodiment, the anti-PD-L1 single-domain antibody (VHH) includes the HCDR1 as shown in SEQ ID NO: 69, the HCDR2 as shown in SEQ ID NO: 96 and the HCDR3 as shown in SEQ ID NO: 71.

In another preferred embodiment, the anti-PD-L1 single-domain antibody (VHH) further includes a humanized FR region.

In another preferred embodiment, the amino acid sequence of the anti-PD-L1 single-domain antibody (VHH) is as shown in SEQ ID NO: 51, SEQ ID NO: 49, SEQ ID NO: 50, SEQ ID NO: 52, SEQ ID NO: 53 or SEQ ID NO: 54.

In another preferred embodiment, the amino acid sequence of VL of the bispecific antibody is as shown in SEQ ID NO: 29, the amino acid sequence of VH is as shown in SEQ ID NO: 31, and the amino acid sequence of the anti-PD-L1 single-domain antibody (VHH) is as shown in SEQ ID NO: 51.

In another preferred embodiment, the amino acid sequence of VL of the bispecific antibody is as shown in SEQ ID NO: 1, the amino acid sequence of VH is as shown in SEQ ID NO: 5, and the amino acid sequence of the anti-PD-L1 single-domain antibody (VHH) is as shown in SEQ ID NO: 51.

In another preferred embodiment, the amino acid sequence of VL of the bispecific antibody is as shown in SEQ ID NO: 16, the amino acid sequence of VH is as shown in SEQ ID NO: 17, and the amino acid sequence of the anti-PD-L1 single-domain antibody (VHH) is as shown in SEQ ID NO: 51.

In another preferred embodiment, the amino acid sequence of VL of the bispecific antibody is as shown in SEQ ID NO: 8, the amino acid sequence of VH is as shown in SEQ ID NO: 13, and the amino acid sequence of the anti-PD-L1 single-domain antibody (VHH) is as shown in SEQ ID NO: 51.

In another preferred embodiment, the amino acid sequence of VL of the bispecific antibody is as shown in SEQ ID NO: 21, the amino acid sequence of VH is as shown in SEQ ID NO: 26, and the amino acid sequence of the anti-PD-L1 single-domain antibody (VHH) is as shown in SEQ ID NO: 51.

In another preferred embodiment, the amino acid sequence of the L chain (VL-L3-CL) of the bispecific antibody is as shown in SEQ ID NO: 63, SEQ ID NO: 59, SEQ ID NO: 61, or SEQ ID NO: 65; and the amino acid sequence of the H chain (VH-L1-CH-L2-VHH) of the bispecific antibody is as shown in SEQ ID NO: 64, SEQ ID NO: 60, SEQ ID NO: 62 or SEQ ID NO: 66.

In another preferred embodiment, the amino acid sequence of the L chain (VL-L3-CL) of the bispecific antibody is as shown in SEQ ID NO: 63 and the amino acid sequence of the H chain (VH-L1-CH-L2-VHH) is as shown in SEQ ID NO: 64.

In another preferred embodiment, the amino acid sequence of the L chain (VL-L3-CL) of the bispecific antibody is as shown in SEQ ID NO: 59 and the amino acid sequence of the H chain (VH-L1-CH-L2-VHH) is as shown in SEQ ID NO: 60.

In another preferred embodiment, the amino acid sequence of the L chain (VL-L3-CL) of the bispecific antibody is as shown in SEQ ID NO: 61 and the amino acid sequence of the H chain (VH-L1-CH-L2-VHH) is as shown in SEQ ID NO: 62.

In another preferred embodiment, the amino acid sequence of the L chain (VL-L3-CL) of the bispecific antibody is as shown in SEQ ID NO: 65 and the amino acid sequence of the H chain (VH-L1-CH-L2-VHH) is as shown in SEQ ID NO: 66.

In another preferred embodiment, the bispecific antibody is a partially or fully humanized antibody.

A second aspect of the present disclosure provides an isolated polynucleotide, in which the polynucleotide encodes the bispecific antibody of the first aspect of the present disclosure.

In another preferred embodiment, the polynucleotide includes a DNA, RNA or cDNA.

A third aspect of the present disclosure provides a vector containing the polynucleotide of the second aspect of the present disclosure.

In another preferred embodiment, the expression vector is selected from: a plasmid and a viral vector.

In another preferred embodiment, the expression vector includes: a bacterial plasmid, a phage, a yeast plasmid, a plant cell virus, a mammalian cell virus such as an adenovirus, an adeno-associated virus AAV, a retrovirus, or other vectors.

The fourth aspect of the present disclosure provides a genetically engineered host cell, said host cell contains the vector of the third aspect of the present disclosure, or the polynucleotide of the second aspect of the present disclosure that is integrated into its genome.

In another preferred embodiment, the host cell includes a prokaryotic cell or a eukaryotic cell.

In another preferred embodiment, the host cell is selected from following group: *Escherichia coli.,* a yeast cell, and a mammalian cell.

A fifth aspect of present disclosure provides a method of preparing the bispecific antibody of the first aspect of the present disclosure, including the steps of:
(i) culturing the host cell of the fourth aspect of the present disclosure under suitable conditions, thereby obtaining a mixture containing the bispecific antibody of the first aspect of the present disclosure;
(ii) purifying and/or separating the mixture obtained in step (i) to obtain the bispecific antibody.

In another preferred embodiment, the purification can be performed by purification and separation via protein A affinity column to obtain the target antibody.

In another preferred embodiment, the purity of the purified and separated target antibody is greater than 95%, greater than 96%, greater than 97%, greater than 98%, greater than 99%, preferably 100%.

The sixth aspect of the present disclosure provides a pharmaceutical composition, the pharmaceutical composition including:
(a) the bispecific antibody of the first aspect or the conjugate of the seventh aspect of the present disclosure; and
(b) a pharmaceutically acceptable carrier.

In another preferred embodiment, the pharmaceutical composition also contains other drugs for treating cancer (or tumor), such as chemotherapeutics.

In another preferred embodiment, the pharmaceutical composition is used for blocking the interaction between PD-1 and PD-L1, and simultaneously binding claudin 18.2 protein.

In another preferred embodiment, the pharmaceutical composition is used for treating cancers (or tumors) that express claudin18.2 protein (i.e. Claudin18.2 positive).

In another preferred embodiment, the pharmaceutical composition is a dosage for injection.

The seventh aspect of the present disclosure provides an immunoconjugate, the immunoconjugate including:
(a) the bispecific antibody of the first aspect of the present disclosure; and
(b) a coupling moiety selected from the group consisting of a detectable label, a drug, a toxin, a cytokine, a radionuclide, or an enzyme.

An eighth aspect of the present disclosure provides the uses of the bispecific antibody of the first aspect of the present disclosure in the preparation of drugs for treating cancers (or tumors), infection or immunoregulatory diseases.

A ninth aspect of the present disclosure provides the uses of the bispecific antibody of the first aspect of the present disclosure in the preparation of drugs for inhibiting tumor growth.

In another preferred embodiment, the cancer or tumor is selected from the group consisting of colorectal cancer, breast cancer, ovarian cancer, pancreatic cancer, gastric cancer, esophageal cancer, prostate cancer, renal cancer, cervical cancer, bone marrow cancer, lymphoma, leukemia, thyroid cancer, endometrial cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.

The main beneficial effects of the present disclosure include: the present disclosure provides a bispecific antibody simultaneously targeting Claudin18.2 and PD-L1, and the bispecific antibody molecule can target Claudin18.2 and PD-L1 with high efficiency. The present disclosure can improve the therapeutic effects on the tumors expressing claudin18.2. The bispecific antibody can not only bind human claudin18.2 protein, but also block the binding of PD-1/PD-L1, it can not only activate NK cells in innate immunity to kill tumor cells, and but also promote the killing effects of killer T lymphocytes in acquired immunity on tumors, thus acquiring a synergistic tumor-killing effect. The bispecific antibody has better anti-tumor effects than the anti-claudin18.2 antibody used alone.

### Brief description of the drawings

FIG. 1 shows a structural schematic diagram of an anti-CLDN18.2/anti-PD-L1 bispecific antibody molecule.
FIG. 2 shows the results for activity identification of the first group humanized Claudin18.2 antibody by ELISA.
FIG. 3 shows the results for activity identification of the second group humanized Claudin18.2 antibodyby ELISA.
FIG. 4 shows the results for activity of the third group humanized Claudin18.2 antibody by ELISA.
FIG. 5 shows the results for activity of the fourth group humanized Claudin18.2 antibody by ELISA.
FIG. 6 shows the results for activity of the fifth group humanized Claudin18.2 antibody by ELISA.
FIG. 7 shows the results for activity of the first group humanized Claudin18.2 antibody by FACS.
FIG. 8 shows the results for activity of the second group humanized Claudin18.2 antibody by FACS.
FIG. 9 shows the results for activity of the third group humanized Claudin18.2 antibody by FACS.
FIG. 10 shows the results for activity of the fourth group humanized Claudin18.2 antibody by FACS.
FIG. 11 shows the results for activity of the fifth group of humanized Claudin18.2 antibody by FACS.
FIG. 12 shows the results of ADCC of the humanized Claudin18.2 antibody.
FIG. 13 shows the results of CDC of the humanized Claudin18.2 antibody.
FIG. 14 shows the *in vivo* pharmacodynamic results of the humanized Claudin18.2 antibody in CB. 17-SCID model of immunodeficient mice.
FIG. 15 shows the ELISA results for binding activity identification of the humanized anti-PD-L1 single-domain antibody.
FIG. 16 shows ELISA results for blocking activity identification of the humanized anti-PD-L1 single-domain antibody.
FIG. 17 shows the results of expression and purification of the first anti-CLDN18.2/anti-PD-L1 bispecific antibody.
FIG. 18 shows the results of expression and purification of the second anti-CLDN18.2/anti-PD-L1 bispecific antibody for the second antibody.
FIG. 19 shows the results of expression and purification of the third anti-CLDN18.2/anti-PD-L1 bispecific antibody.
FIG. 20 shows the results of expression and purification of the fourth anti-CLDN18.2/anti-PD-L1 bispecific antibody.
FIG. 21 shows the ELISA results for binding activity identification of the anti-CLDN18.2/anti-PD-L1 bispecific antibody.
FIG. 22 shows the ELISA results for blocking activity of the anti-CLDN18.2/anti-PD-L1 bispecific antibody to PD-L1/PD-1.
FIG. 23 shows the ELISA results for blocking activity of the anti-CLDN18.2/anti-PD-L1 bispecific antibody to PD-L1/CD80.
FIG. 24 shows the ELISA results for binding activity of CLDN18.2 to anti-CLDN18.2/anti-PD-L1 bispecific antibody.
FIG. 25 shows the identification of the functional activity of anti-CLDN18.2/anti-PD-L1 bispecific antibody (concentration-dependent results of the cytokine IFNγ produced after T cell activation by candidate molecule CHO14 in mixed lymphocyte reaction MLR).
FIG. 26 shows the identification of the functional activity of anti-CLDN18.2/anti-PD-L1 bispecific antibody (concentration-dependent results of the cytokine IL-2 produced after T cell activation by candidate molecule CHO14 in mixed lymphocyte reaction MLR).
FIG. 27 shows the results of PBMC-mediated cell-killing experiment of the anti-CLDN18.2/anti-PD-L1 bispecific antibody.
FIG. 28 shows the results of *in vivo* pharmacodynamics evaluation of the anti-CLDN18.2/anti-PD-L1 bispecific antibody in immune target humanized transgenic mouse C57BL/6-hPDL1 model MC38-hPDL1-mClaudin18.2.
FIG. 29 shows the results of *in vivo* pharmacodynamics evaluation of the anti-CLDN18.2/anti-PD-L1 bispecific antibody in immune system humanized mouse PBMC engrafted-NCG model HCC827-hClaudin18.2.
FIG. 30 shows the evaluation of *in vivo* synergistic pharmacodynamics of the anti-CLDN18.2/anti-PD-L1 bispecific antibody in C57BL/6 model MC38-hPDL1-mClaudin18.2.
FIG. 31 shows the evaluation of *in vivo* synergistic pharmacodynamics of the anti-CLDN18.2/anti-PD-L1 bispecific antibody in C57BL/6 model MC38-hPDL1-mClaudin18.2.

### Detailed description of the preferred embodiment

Through extensive and in-depth research and a large number of screenings, the inventors obtained anti-CLDN18.2 antibody and anti-PD-L1 single-domain antibody with high specificity and affinity; and humanization and genetic recombination were carried out on this basis, thus obtaining a bispecific antibody simultaneously targeting human Claudin18.2 and human PD-L1. *In vitro* experiments proved that the bispecific antibody of the present disclosure can specifically bind human Claudin18.2 and human PD-L1 molecules, and kill human lung cancer cells that recombinantly express Claudin 18.2 and naturally express PD-L1. *In vivo* pharmacodynamics experiments proved that the bispecific antibody of the present disclosure had synergistic effects, and shows superior antitumor activity in humanized mouse model as compared with Claudin18.2 monoclonal antibody and PD-L1 monoclonal antibody. The present disclosure is accomplished on this basis.

### Terms

Certain terms are firstly defined in order to make this disclosure be more easily understood. As used in the present application, each of the following terms shall have the meanings given as follows unless expressly stated otherwise herein. Other definitions are stated throughout the application.

As used herein, the terms "bispecific antibody of the present disclosure", "double antibody of the present disclosure" and "bispecific antibody against claudin18.2/ PD-L1" have the same meaning, and all refer to bispecific antibodies that specifically recognize and bind claudin18.2 and PD-L1.

As used herein, the term "antibody" or "immunoglobulin" is heterotetrameric glycoprotein about 150,000 Daltons with the same structural characteristics, which consists of two identical light chains (L) and two identical heavy chains (H). Each light chain is connected to the heavy chain via a covalent disulfide bond, but the number of disulfide bonds between the heavy chains of different immunoglobulin isoforms is different. Each heavy chain and light chain also have intra-chain disulfide bonds in regular intervals. There are two types of light chains: λ(1) and κ(k). There are main five types (or isoforms) of heavy chains that determine the functional activity of antibody molecules: IgM, IgD, IgG, IgA and IgE. Each chain contains a different sequence domain. The light chain includes two domains or regions: a variable domain (VL) and a constant domain (CL). The heavy chain includes four domains: a heavy chain variable region (VH) and three constant regions (CH1, CH2 and CH3, collectively referred to as CH). The variable regions of both light chain (VL) and heavy chain (VH) determine the binding recognition and specificity of antigen. The constant domain of light chain (CL) and constant region of heavy chain (CH) confer important biological properties such as antibody chain binding, secretion, transplacental mobility, complement binding and Fc receptor (FcR) binding. Fv fragment is the N-terminal moiety of immunoglobulin Fab fragment and consists of the variable moieties a light chain and a heavy chain. The specificity of antibody depends on the structural complementation between the antibody binding sites and the antigenic determinants. The antibody binding sites are composed of residues mainly from highly variable regions or complementary determining regions (CDRs). Occasionally, residues from non-highly variable or framework regions (FR) affect the overall domain structure and thus the binding sites. Complementary regions or CDRs refer to amino acid sequences that jointly define the binding affinity and the specificity of the natural Fv region of the natural immunoglobulin binding site. The light chain and heavy chain of immunoglobulin each have three CDRs, which are called LCDR1 (CDR1-L), LCDR2 (CDR2-L), LCDR3(CDR3-L) and HCDR1 (CDR1-H), HCDR2 (CDR2-H) and HCDR3 (CDR3-H), respectively. The conventional antibody-antigen binding sites therefore include six CDRs, including a collection of CDRs from the V regions of each heavy chain and light chain.

As used herein, the terms "single-domain antibody", "VHH" and "nanobody" have the same meanings, and refer to cloning the variable region of the heavy chain of the antibody, and constructing a nanobody consisting of only one variable region of the heavy chain (VHH), which is the smallest antigen-binding fragment with complete functions. Usually, the antibody with natural deletion of light chain and heavy chain constant region 1(CH1) is obtained first, and then the variable region of antibody heavy chain is cloned to construct a nanobody consisting of only one variable region of heavy chain (VHH).

As used herein, the term "variable" means that some moieties of the variable region of an antibody are different in sequence, which forms the binding and specificity of various specific antibodies to their specific antigens. However, variability is not evenly distributed in the whole variable region of the antibody. It is concentrated in three segments of variable regions of light chain and heavy chain, which are called complementary determining regions (CDRs) or hypervariable regions. The relatively conservative moiety of the variable region is called frame region (FR). Four FR regions are contained in each variable region of natural heavy chain and light chain, which are roughly inβ-folded configuration, connected by three CDRs forming a connecting ring, and in some cases can form a partiallyβ folded structure. The CDRs in each chain are tightly attached together through the FR regions and form the antigen-binding sites of the antibody together with the CDRs in the other chain (see Kabat *et al.,* NIH Publ. No. 91-3242, Volume I, pages 647-669 (1991)). Constant regions are not directly involved in the binding of antibodies to antigens, but they show different effector functions, for example, involve in the antibody-dependent cytotoxicity of the antibody.

As used herein, the term "framework region" (FR) refers to the amino acid sequences inserted between CDRs, that is, those moieties of the variable regions of light chain and heavy chain of immunoglobulins that are relatively conserved among different immunoglobulins in a single species. The light chains and heavy chains of an immunoglobulin each have four FRs, which are called FR1-L, FR2-L, FR3-L, FR4-L and FR1-H, FR2-H, FR3-H and FR4-H, respectively. Accordingly, the light chain variable domain can be called (FR1-L)-(CDR1-L)-(FR2-L)-(CDR2-L)-(FR3-L)-(CDR3-L)-(FR4-L) and the heavy chain variable domain can be expressed as (FR1-H)-(CDR1-H)-(FR2-H)-(CDR2-H)-(FR3-H)-(CDR3-H)-(FR4-H). Preferably, the FRs of the present disclosure are FRs of a human antibody or derivatives thereof, and the derivatives of the FRs of human antibody are substantially identical to the FRs of naturally occurring human antibody, that is, the sequence identity reaches 85%, 90%, 95%, 96%, 97%, 98% or 99%.

Upon knowing the amino acid sequence of CDR, those skilled in the art can easily determine the frame regions FR1-L, FR2-L, FR3-L, FR4-L and/or FR1-H, FR2-H, FR3-H and FR4-H.

As used herein, the term "humanized antibody" is an antibody molecule derived from a non-human antibody capable of binding to a target antigen, the target antigen having one or more complementarity determining regions (CDRs) of non-human origin and a framework region derived from human immunoglobulin molecules. Generally, the frame residues in the human frame region will be replaced by corresponding residues from a CDR donor antibody so as to change, preferably improve, the binding to antigens. These frame substitutions can be identified by a well-known method in the art, for example, by simulating the interaction between CDRs and frame residues to identify frame residues that play an important role in antigen binding, and by sequence comparison to identify abnormal frame residues at specific positions. The antibody can be humanized using various well-known techniques in the art, such as CDR grafting (EP 239,400, PCT publication WO 91/09967, U.S. Pat. Nos.5,225,539; 5,530,101 and 5,585,089), veneering or resurfacing (EP 592,106, EP 519,596, Padlan, Molecular Immunology28(4/5):489-498(1991), Studnicka et al., Protein Engineering 7(6):805-814(1994), Roguska et al., Proc.Natl.Sci.USA 91:969-973(1994)), and chain replacement (U.S.Pat.No.5,565,332), the entire contents of which are incorporated herein by reference.

As used herein, the term "human frame region" is a frame region that is substantially identical (about 85% or more, specifically 90%, 95%, 97%, 99% or 100% identical) to the frame region of a naturally occurring human antibody.

As used herein, the term "linker" refers to one or more amino acid residues inserted into an immunoglobulin domain so as to provide sufficient mobility for the domains of the light chain and the heavy chain to fold into an immunoglobulin having exchanged double variable regions.

The present disclosure includes not only intact antibody, but also the fragments of an antibody with immunological activity or a fusion protein formed by an antibody and other sequences. Therefore, the present disclosure also includes the fragments, derivatives and analogs of the antibody.

As used herein, the terms "fragments", "derivatives" and "analogs" refer to a polypeptide that substantially maintains the same biological function or activity as that of the antibody of the present disclosure. The polypeptide fragments, derivatives or analogs of the present disclosure may be (i) a polypeptide in which one or more conservative or non-conservative amino acid residues (preferably conservative amino acid residues) are substituted, and such substituted amino acid residues may or may not be encoded by a genetic code, or (ii) a polypeptide with a substituent group in one or more amino acid residues, or (iii) a polypeptide formed by fusion of a mature polypeptide to another compound (such as a compound that prolongs the half-life of a polypeptide, e.g., polyethylene glycol), or (iv) a polypeptide formed by fusing an additional amino acid sequence to this polypeptide sequence (e.g., a leader sequence or a secretion sequence, a sequence used to purify this polypeptide or a proteinogen sequence, or a fusion protein formed with a 6His tag). According to the teachings herein, these fragments, derivatives and analogs are well known to those skilled in the art.

The antibody of the present disclosure refers to a double antibody with the activities of binding claudin18.2 and PD-L1 protein. The term also includes the variation forms of the polypeptides having the same function as the antibody of the present disclosure and containing the same CDR regions. These variation forms include (but are not limited to) the deletion, insertion and/or substitution of one or more (usually 1-50, preferably 1-30, more preferably 1-20, and most preferably 1-10) amino acids, and the addition of one or more (usually within 20, preferably within 10, and more preferably within 5) amino acids to the C terminal and/or the N terminal. For example, in this art, when amino acids with the similar or alike properties are used for substitution, the functions of the protein usually would not be changed. Also, for example, the addition of one or more amino acids to the C-terminal and/or N-terminal usually would not change the functions of the protein.

The variation forms of the polypeptides include homologous sequences, conservative variants, allelic variants, natural mutants, induced mutants, the proteins encoded by DNA that can hybridize with the encoding DNA of the antibody of the present disclosure under high or low stringency conditions, and the polypeptides or proteins obtained using the antiserum against the antibody of the present disclosure.

Among them, "conservative variants" refer to that, as compared with the amino acid sequence of the antibody of the present disclosure, at most 10, preferably at most 8, more preferably at most 5, and most preferably at most 3 amino acids are replaced by the amino acids with similar or alike properties to form a polypeptide (especially the framework region is replaced by the amino acids with similar or alike properties to form a polypeptide). These conservative variant polypeptides are best produced by the amino acid substitutions according to Table A.

**Table A**

| Initial residue | Representative substitution | Preferred sub stitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present disclosure also provides polynucleotide molecules encoding the antibodies described above or fragments thereof or fusion proteins thereof. The polynucleotides of the present disclosure may be in the form of DNA or the form of RNA. The forms of DNA include cDNA, genomic DNA or artificially synthetic DNA. DNA can be single-stranded or double-stranded. DNA can be a coding strand or a non-coding strand.

The polynucleotides encoding the mature polypeptides of the present disclosure include: coding sequences only encoding mature polypeptides; coding sequence of mature polypeptide and various additional coding sequences; coding sequences (and optional additional coding sequences) of mature polypeptides and non-coding sequences.

The term "polynucleotide encoding a polypeptide" may include the polynucleotide encoding the polypeptide, or may also include additional coding and/or non-coding sequences.

The present disclosure also relates to a polynucleotide which hybridizes with the above sequences and has at least 50%, preferably at least 70%, more preferably at least 80% identity between the two sequences. In particular, the present disclosure relates to a polynucleotide which can be hybridized with the polynucleotide of the present disclosure under stringent conditions. In the present disclosure, "stringent conditions" refer to: (1) hybridization and elution at a relatively low ionic strength and a relatively high temperature, such as 0.2×SSC, 0.1%SDS, 60°C; or (2) a denaturant, such as 50%(v/v) formamide, 0.1% calf serum/0.1% Ficoll (42°C, etc.) are added during hybridization; or (3) hybridization only occurs when the identity between the two sequences is at least 90%, more preferably 95%. Moreover, the polypeptide encoded by the hybridizable polynucleotide has the same biological functions and activities as that of the mature polypeptide.

The full-length nucleotide sequence of the antibody of present disclosure or its fragments can usually be obtained by a PCR amplification method, a recombination method or an artificial synthesis method. A feasible method is to synthesize the relevant sequence using an artificial synthesis method, especially when the fragment length is relatively short. Usually, a fragment having a very long sequence can be obtained by firstly synthesizing multiple small fragments and then ligating them. In addition, the coding sequence of the heavy chain and the expression tag (such as 6His) can be fused together to form a fusion protein.

Once a relevant sequence is obtained, the recombination method can be used to obtain the relevant sequence in large quantities. This is usually done by cloning it into a vector, then transferring it into a cell, and then isolating the relevant sequence from the proliferated host cells by conventional methods. The biomolecules (nucleic acids, proteins, etc.) involved in the present disclosure include the biomolecules that exist in an isolated form.

Currently, the DNA sequence encoding the protein (or a fragment or derivative thereof) of the present disclosure can be obtained completely through chemical synthesis. The DNA sequence can then be introduced into various existing DNA molecules (or such as vectors) and cells known in the art. In addition, mutations can also be introduced into the protein sequence of the present disclosure by chemical synthesis.

The present disclosure also relates to a vector containing the appropriate DNA sequence described above and an appropriate promoter or control sequence. These vectors can be used to transform an appropriate host cell so that it can express a protein.

The host cell can be a prokaryotic cell such as a bacterial cell; or a lower eukaryotic cell such as a yeast cell; or a higher eukaryotic cell, such as a mammalian cell. Representative examples are: Escherichia coli and Streptomyces; bacterial cells of Salmonella typhimurium; fungi such as yeast; insect cells of Drosophila S2 or Sf9; animal cells of CHO, COS7, 293 cells, etc.

The transformation of the host cell using recombinant DNA can be performed by conventional techniques well known to those skilled in the art. When the host is a prokaryote such as Escherichia coli, a competent cell which is capable of absorbing DNA can be harvested after the exponential growth phase and treated by CaCl₂ method, in which the relevant steps used are well known in the art. Another method is to use MgCl₂. If necessary, transformation can also be performed by a method of electroporation. When the host is a eukaryote, the following DNA transfection methods can be selected: the calcium phosphate co-precipitation method, and the conventional mechanical methods such as microinjection, electroporation, liposome packaging, etc.

The obtained transformants can be cultured by conventional methods to express the polypeptide encoded by the gene of the present disclosure. Depending on the host cell used, the medium used in the culture can be selected from various conventional culture media. The culture is carried out under the conditions suitable for the growth of the host cell. When the host cell grows to an appropriate cell density, a suitable method (such as temperature conversion or chemical induction) is used to induce the selected promoter, and the cell is further cultured for a period of time.

The recombinant polypeptide in the above method can be expressed in the cell or on the cell membrane or secreted out of the cell. If necessary, the recombinant protein can be separated and purified through various separation methods using its physical, chemical and other properties. These methods are well known to those skilled in the art. Examples of these methods include, but are not limited to: conventional renaturation treatment, treatment with protein precipitation agent (salting out method), centrifugation, cell breaking via osmosis, ultra-treatment, ultra-centrifugation, molecular sieve chromatography (gel filtration), adsorption chromatography, ion exchange chromatography, high performance liquid chromatography (HPLC) and various other liquid chromatography techniques and the combinations of these methods.

The antibody of the present disclosure can be used alone, or combined or coupled with a detectable marker (for diagnostic purposes), a therapeutic agent, a modified part of PK (protein kinase) or a combination of any of the above.

The detectable label for diagnostic purposes includes, but are not limit to, a fluorescent or luminescent label, a radioactive label, a MRI (magnetic resonance imaging) or CT (electronic computer X-ray tomography technique) contrast agent, or an enzyme capable of producing a detectable product.

Therapeutic agents that can bind or couple with the antibody of the present disclosure include, but are not limited to: 1. a radionuclide; 2. a biological poison; 3. a cytokine such as IL-2; 4. a gold nanoparticle/nanorod; 5. a virus particle; 6. a liposome; 7. a nanometer magnetic particle; 8. a prodrug-activating enzyme (e.g., DT-diaphorase (DTD) or a biphenyl hydrolase-like protein (BPHL)); 10. a chemotherapeutic agent (e.g., cisplatin) or nanoparticles in any forms, etc.

As known to those skilled in the art, immunoconjugates and fusion expression products include the conjugates formed by the binding of a drug, a toxin, a cytokine, a radionuclide, an enzyme and other diagnostic or therapeutic molecules with the antibody of the present disclosure or fragments thereof.

### Bispecific antibody of the present disclosure

The present disclosure provides a bispecific antibody targeting human claudin18.2 and human PD-L1 protein, which includes an anti-human claudin18.2 antibody moiety and an anti-PD-L1 antibody moiety.

### (1) anti-human claudin18.2 antibody moiety

The anti-human claudin18.2 antibody moiety in the bispecific antibody of the present disclosure is obtained by humanized modification of the mouse-derived anti-claudin18.2 antibody. By means of aligning the germline gene database of the heavy and light chain variable regions of IMGT human antibody using MOE software, the germline genes of the heavy and light chain variable regions having high homology with QP190191, QP192193, QP199200, QP201202 and QP207208 were selected respectively as templates, and the CDRs of a mouse-derived antibody were grafted into the corresponding humanized templates, and a variable region sequence in an order of FR1-CDR1-FR2-CDR2-FR3-CDR3-FR4 was formed. Then, some important amino acid residues are selected for reverse mutation combinations.

The heavy chain variable regions (VHs) of the mouse-derived anti-claudin18.2 antibody have the following amino acid sequences, respectively, and the CDRs are shown in the underlined parts of the sequences:
>QD208 SEQ ID NO.46 
EVQLQQSGPELVKPGASVKMSCKASGYTFTSYIMHWVKQKPGQGLEWI
GYINPYNDGTKYNEKFKGKATLTSDKSSSTVYMELSSLTSEDSAVYCCARLGF
TTRNAMDYWGQGTSVTVSS
>QP191 SEQ ID NO.38 
EVKLVESGGGLVKPGGSLKLSCAASGFTFSNYAMSWVRQTPEKRLEWVA
SIISGGRTYYLDSEKGRFTISRDNARNNLYLQMSSLRSEDTAMYYCTRIYYGNS
 FDYWGQGTTLTVSS >QD193 SEQ ID NO.40 
QVQLQQSGAELVRPGSSVKISCKASGYAFSSYWMNWVKQRPGQGLEWI
GQIYPGNGDTTYNGKFKGQATLTADKSSSTVYMQLSSLTSEDSAVYFCARFVK
GNAMDYWGQGTSVTVSS
 >QD200 SEQ ID NO.42 
DVQLVESGGGLVQPGGSRKLSCAASGFTFSSFGMHWVRQAPEKGLEWV
AYISSGSNSIYYVDTVKGRFTISRDNPKNTLFLQMTSLKSEDTAMYYCARNAY
YGNSFDYWGQGTTLTVSS
 >QD202 SEQ ID NO.44

EVQLQQSGPELVKPGASVKMSCKASGYTFTNYFVHWVKQKPGQGLEWI
GYINPYNDDTKYNEKFKGKATLTSDKSSSTAYMDLSSLTSEDSAVYYCLSLRFF
AYWGQGTLVTVSA

The light chain variable regions of the mouse-derived anti-claudin18.2 antibody have the following amino acid sequences, respectively, and the CDRs are shown in the underlined parts of the sequences:
>QD207 SEQ ID NO: 45 
DIVMTQSPSSLSVSAGEKVTMNCKSSQSLLNSGNQKNYLAWYQQKPGQ
PPKLLIYGASTRESGVPDRFTGSGSGTDFTLTISSVQAEDLAVYYCQNDHSYPF
TFGSGTKLEIK
>QD190 SEQ ID NO: 37 
DIVMTQSPSSQTVTAGEKVTMSCKSSQSLLNSGNQKNYLTWYQQKPGQP
PKLLIYWASTRESGVPDRFTGSGSGTDFTLTISNMQAEDLAVYYCQNDYSYPF
TFGSGTKLEIK
>QD192 SEQ ID NO: 39 
DIVMTQSPSSLTVTAGEKVTMSCKSSQSLLNSGNQKNYLTWYQQNPGQP
PKMLIYWASTRESGVPDRFTGSGSGIDFSLTISSVQAEDLALYYCQNAYSYPFT
FGSGTKLEIK
>QD199 SEQ ID NO: 41 
DIVMTQSPSSLTVTAGEKVTMSCKSSQSLLNSGNQKNYLTWYQQKPGQP
PKLLIYWASTRESGVPDRFTGSGSGTVFTLTISSVQAEDLAVYFCQNNYYYPLT
FGAGTKLELK
>QD201 SEQ ID NO: 43

DIVMTQSPSSLTVTAGEKVTMSCKSSQSLLNSGNQKNYLTWYQQKPGQA
PKLLIYWASTRESGVPDRFIGSGSGTDFTLTISHVQAEDLAVYFCQNDYSYPLT
FGAGTNLELK

The CDRs (underlined parts) of the light and heavy chain variable regions of the above antibody are listed in Table B.

**Table B**

| Mous e deriv ed | Humani zed VH/VL plasmid number | | CDR1 | CDR2 | CDR3 |
|---|---|---|---|---|---|
| QD2 08 | QD1463 | V | SYIMH SEQ ID NO: 93 | YINPYNDGTKYNE KFKG SEQ ID NO: 94 | LGFTTRNA MDY SEQ ID NO: 95 |
| | QD1464 | H | | | |
| | QD1465 | | | | |
| | QD1466 | | | | |
| QD2 07 | QD1460 | V | KSSQSLLNSGNQK NYLA SEQ ID NO: 90 | GASTRES SEQ ID NO: 91 | QNDHSYPF T SEQ ID NO: 92 |
| | QD1461 | L | | | |
| | QD1462 | | | | |
| QD1 91 | QD1436 | V | NYAMS SEQ ID NO: 75 | SIISGGRTYYLDSE KG SEQ ID NO: 76 | IYYGNSFD Y SEQ ID NO: 77 |
| | QD1437 | H | | | |
| | QD1438 | | | | |
| QD1 90 | QD1433 | V | KSSQSLLNSGNQK NYLT SEQ ID NO: 72 | WASTRES SEQ ID NO: 73 | QNDYSYPF T SEQ ID NO: 74 |
| | QD1434 | L | | | |
| | QD1435 | | | | |
| QD1 93 | QD1443 | V | SYWMN SEQ ID NO: 79 | QIYPGNGDTTYNG KFKG SEQ ID NO: 80 | FVKGNAMD Y SEQ ID NO: 81 |
| | QD1444 | H | | | |
| | QD1445 | | | | |
| QD1 92 | QD1439 | V | KSSQSLLNSGNQK NYLT SEQ ID NO: 72 | WASTRES SEQ ID NO: 73 | QNAYSYPF T SEQ ID NO: 78 |
| | QD1440 | L | | | |
| | QD1441 | | | | |
| | QD1442 | | | | |
| QD2 00 | QD1449 | V | SFGMH SEQ ID NO: 83 | YISSGSNSIYYVDT VKG SEQ ID NO: 84 | NAYYGNSF DY SEQ ID NO: 85 |
| | QD1450 | H | | | |
| | QD1451 | | | | |
| QD1 99 | QD1446 | V | KSSQSLLNSGNQK NYLT SEQ ID NO: 72 | WASTRES SEQ ID NO: 73 | QNNYYYPL T SEQ ID NO: 82 |
| | QD1447 | L | | | |
| | QD1448 | | | | |
| QD2 02 | QD1456 | V | NYFVH SEQ ID NO: 87 | YINPYNDDTKYNE KFKG SEQ ID NO: 88 | LSLRFFAY SEQ ID NO: 89 |
| | QD1457 | H | | | |
| | QD1458 | | | | |
| | QD1459 | | | | |
| QD2 01 | QD1452 | V | KSSQSLLNSGNQK NYLT SEQ ID NO: 72 | WASTRES SEQ ID NO: 73 | QNDYSYPL T SEQ ID NO: 86 |
| | QD1453 | L | | | |
| | QD1454 | | | | |
| | QD1455 | | | | |

### (2) anti-PD-L1 antibody moiety

The anti-human PD-L1 antibody moiety in the bispecific antibody of the present disclosure is obtained by the humanized modification of an anti-PD-L1 single-domain antibody (anti-PD-L1 nanobody). By means of aligning the germline gene database of heavy and light chain variable regions of IMGT human antibody using MOE software, the germline genes of heavy and light chain variable regions having high homology with QP1162 and QP1166 were selected respectively as templates, and the CDRs of a single-domain antibody were grafted into the corresponding humanized templates (such as IGHV3-23 germline and J-region IGHJ4*01), and a variable region sequence in an order of FR1-CDR1-FR2-CDR2-FR3-CDR3-FR4 was formed. Then, some important amino acid residues are selected for reverse mutation combinations.

The amino acid sequences of the anti-PD-L1 single-domain antibody are shown as follows, and the CDRs are shown in the underlined parts of the sequences:
>QD1162 SEQ ID NO: 55 
QVQLVESGGGSVQSGGSLRLSCAASGFTYGTYAMSWFRQAPGKEREGV
ACIDIYGRASYTDPVKGRFTISQDNAKNTLYLQMNSLKPEDTAMYYCAARDF
GYCTASWVHEGFSRYWGQGTQVTVSS
>QD1166 SEQ ID NO: 56

QVQLVESGGDSVQPGGSLRLSCAASGFTYGTYAMSWFRQAPGKEREGV
ACIDIYGRTSYTDPVKGRFTISQDNAKNTLYLQMNSLKPEDTAMYYCAARDF
GYCTASWVHEGFSRYWGQGTQVTVSS

The CDR (underlined part) regions of the above single-domain antibody are summarized as shown in the following table.

| | Plasmid number of humanize d VHH | CDR1 | CDR2 | CDR3 |
|---|---|---|---|---|
| QD116 2 | QD320 | TYAM S SEQ ID NO: 69 | CIDIYGRASYTDPV KG SEQ ID NO: 70 | ARDFGYCTASWVHEGFS RY SEQ ID NO: 71 |
| | QD321 | | | |
| | QD322 | | | |
| QD116 6 | QD323 | TYAM S SEQ ID NO: 69 | CIDIYGRTSYTDPVK G SEQ ID NO: 96 | ARDFGYCTASWVHEGFS RY SEQ ID NO: 71 |
| | QD324 | | | |
| | QD325 | | | |

As used herein, the terms "anti-PD-L1 single-domain antibody" and "anti-PD-L1 nanobody" can be used interchangeably, both of which refer to the antibodies that have natural deletion of light chain, only contain one heavy chain variable region (VHH) and two conventional CH2 and CH3 regions, and target PD-L1 molecules.

As used herein, the term "affinity" is theoretically defined by the equilibrium association between an intact antibody and an antigen. The affinity of the bispecific antibody of the present disclosure can be evaluated or measured by KD values (dissociation constants) (or other assay methods), such as biofilm layer interference techniques (Bio-layer interferometry BLI), as measured using FortebioRed96 instrument.

### Pharmaceutical composition

A composition is provided, which includes: Preferably, the composition is a pharmaceutical composition, which includes the antibody described above or an active fragment or a fusion protein thereof, and a pharmaceutically acceptable carrier. In general, these substances can be prepared into a nontoxic, inert and pharmaceutically acceptable aqueous carrier medium, in which its pH is generally 5-8, preferably the pH is about 6-8, although the pH values can be changed with the nature of the substances to be prepared and the diseases to be treated. The prepared pharmaceutical composition can be administered by conventional routes, including (but not limited to) intratumoral, intraperitoneal, intravenous or local administration.

The pharmaceutical composition of the present disclosure can be directly used for binding claudin18.2 and/or PD-L1 protein molecules, so it can be used for treating tumors. In addition, it can also be used in combination with other therapeutic agents.

The pharmaceutical composition of the present disclosure contains a safe and effective amount (such as 0.001-99wt%, preferably 0.01-90wt%, more preferably 0.1-80 wt%) of the above bispecific antibody (or its conjugate) of the present disclosure and a pharmaceutically acceptable carrier or excipient. Such carriers include, but are not limited to, saline, buffer, glucose, water, glycerol, ethanol, and combinations thereof. The pharmaceutical preparations should match the modes of administration. The pharmaceutical composition of the present disclosure can be prepared into injection forms, for example, it can be prepared with normal saline or aqueous solution containing glucose and other adjuvants by conventional methods. The pharmaceutical composition such as an injection and a solution should be manufactured under aseptic conditions. The dosage of the active ingredient is a therapeutically effective amount, for example, about 10 µg/kg body weight to about 50 mg/kg body weight per day. In addition, the polypeptide of the present disclosure can also be used together with other therapeutic agents.

When the pharmaceutical composition is used, a safe and effective amount of the immunoconjugate is administered to mammals, wherein the safe and effective amount is usually at least about 10 µg/kg body weight, and in most cases, it does not exceed about 50 µg/kg body weight, preferably the dose is about 10 µg/kg body weight to about 10 mg/kg body weight. Of course, for a specific dosage, the route of administration, the health status of patients and other factors, which are within the skill of a skilled physician, should also be taken into account.

### Use

The present disclosure also provides the uses of the antibody of the present disclosure, and relates to the uses thereof in preparing drugs for treating cancers (or tumors), infections or immunoregulatory diseases. A preferred use is to treat cancers (or tumors).

The main advantages of the present disclosure include:
(1) The present disclosure provides a bispecific antibody simultaneously targeting human Claudin18.2 and human PD-L1, in which the bispecific antibody molecule can efficiently target human Claudin18.2 and human PD-L1.
(2) The bispecific antibody of the present disclosure can improve the therapeutic effects on tumors expressing claudin18.2. The bispecific antibody can not only bind human claudin18.2 protein, but also block the binding of PD-1/PD-L1, it can not only activate NK cells in innate immunity to kill tumor cells, and but also promote the killing effects of killer T lymphocytes in acquired immunity on tumors; therefore, the bispecific antibody has better anti-tumor effects than the anti-claudin18.2 antibody alone.
(3) The bispecific antibody of the present disclosure has synergistic effects.

The technical scheme of the present disclosure will be described in detail with reference to the following specific embodiments.

The experimental methods for which specific conditions are not indicated in the following embodiments are in accordance with conventional conditions or those suggested by the manufacturers of raw materials or commodities. Alternatively, the experimental methods recorded in biotechnology textbooks such as Molecular Cloning, Laboratory Manual, Cold Spring Harbor Laboratory, Modern Molecular Biology Method, Cell Biology, etc. are carried out. The reagents without specific sources are conventional reagents purchased through commercial routes.

### Example 1 Humanization of hybridoma monoclonal anti-Claudin18.2 antibodies

By means of aligning the germline gene database of the heavy and light chain variable regions of an IMGT human antibody using MOE software, the germline genes of the heavy and light chain variable regions having high homology with QP190191, QP192193, QP199200, QP201202 and QP207208 were selected respectively as templates, and the CDRs of a mouse-derived antibody were grafted into the corresponding humanized templates, and a variable region sequence in an order of FR1-CDR1-FR2-CDR2-FR3-CDR3-FR4 was formed. Then, some important amino acid residues are selected for reverse mutation combinations. Among them, the amino acid residues were identified and annotated by Kabat numbering system.

### 1. Construction of humanized anti-Claudin18.2 antibodies

Primer PCR was designed to construct VH/VK gene fragments of various humanized antibodies, and then homologous recombination was carried out with expression vector pQD having signal peptide and constant region gene (CH1-FC/CL) fragments to construct a full-length antibody expression vector VH-CH1-FC-pQD/VK-CL-pQD.

The online software DNAWorks (v3.2.2)(http://helixweb.nih.gov/dnaworks/) was used to design a number of primers to synthesize VH/VK containing the gene fragment needed for recombination: 5'-30bp signal peptide+VH/VK+30bp CH1/CL-3'. According to the operating instructions of Primer STAR GXL DNA polymerase of TaKaRa Company, VH/VK containing gene fragment needed for recombination was obtained by two-step PCR amplification using the primers designed above. Construction and enzyme digestion of expression vector pQD (having signal peptide and constant region gene (CH1-FC/CL) fragment): restriction enzyme BsmBI, as well as the different characteristics of the recognition sequence from that of the digestion sites were used to design and construct the expression vector pQD (having signal peptide and constant region gene (CH1-FC/CL) fragment). The vector was digested using BsmBI enzyme, and the gel was cut and recovered for later use. The recombinantly-constructed expression vector VH-CH1-FC-pQD/VK-CL-pQD.VH/VK containing the gene fragment required for recombination and the expression vector pQD recovered by BsmBI enzyme digestion were added into DH5H competent cells at a ratio of 3:1, then subjected to ice bath at 0°C for 30 min, heat shock at 42°C for 90 s, followed by adding 5 times ofLB medium, incubating at 37°C for 45 min, coating LB-Amp plate, culturing overnight at 37°C, and picking single clones for sequencing to obtain individual clones of interest.

The humanization design for the sequences of the light and heavy chain variable regions of individual clones and the protein expression numbers are shown below; in this table, kappa light chain constant region CL (SEQ ID NO: 67) is employed for all antibody light chains, and human IgG1 constant region (SEQ ID NO: 68) is employed for the antibody heavy chains:

**Table 1: sequences of light and heavy chain variable regions and protein expression numbers of humanization design**

| | Protein number | Heavy chain plasmid number | Sequence number | | Light chain plasmid number | Sequence number |
|---|---|---|---|---|---|---|
| QP1901 91 humaniz ation design | QP14361 433 | QD1436 | SEQ ID NO: 04 | | QD1433 | SEQ ID NO: 01 |
| | QP14361 434 | QD1436 | SEQ ID NO: 04 | | QD1434 | SEQ ID NO: 02 |
| | QP14361 435 | QD1436 | SEQ ID NO: 04 | | QD1435 | SEQ ID NO: 03 |
| | QP14371 433 | QD1437 | SEQ ID NO: 05 | | QD1433 | SEQ ID NO: 01 |
| | QP14371 434 | QD1437 | SEQ ID NO: 05 | | QD1434 | SEQ ID NO: 02 |
| | QP14371 435 | QD1437 | SEQ ID NO: 05 | | QD1435 | SEQ ID NO: 03 |
| | QP14381 433 | QD1438 | SEQ ID NO: 06 | | QD1433 | SEQ ID NO: 01 |
| | QP14381 434 | QD1438 | SEQ ID NO: 06 | | QD1434 | SEQ ID NO: 02 |
| | QP14381 435 | QD1438 | SEQ ID NO: 06 | | QD1435 | SEQ ID NO: 03 |

| | Protein number | Heavy chain plasmid number | Sequence number | | Light chain plasmid number | Sequence number |
|---|---|---|---|---|---|---|
| QP192193 Humani zation design | QP14431 439 | QD 1443 | SEQ ID NO: 11 | | QD1439 | SEQ ID NO: 07 |
| | QP14431 440 | QD 1443 | SEQ ID NO: 11 | | QD1440 | SEQ ID NO: 08 |
| | QP14431 441 | QD1443 | SEQ ID NO: 11 | | QD1441 | SEQ ID NO: 09 |
| | QP14431 442 | QD1443 | SEQ ID NO: 11 | | QD1442 | SEQ ID NO: 10 |
| | QP14441 439 | QD1444 | SEQ ID NO: 12 | | QD1439 | SEQ ID NO: 07 |
| | QP14441 440 | QD1444 | SEQ ID NO: 12 | | QD1440 | SEQ ID NO: 08 |
| | QP14441 441 | QD1444 | SEQ ID NO: 12 | | QD1441 | SEQ ID NO: 09 |
| | QP14441 442 | QD1444 | SEQ ID NO: 12 | | QD1442 | SEQ ID NO: 10 |
| | QP14451 439 | QD1445 | SEQ ID NO: 13 | | QD1439 | SEQ ID NO: 07 |
| | QP14451 440 | QD1445 | SEQ ID NO: 13 | | QD1440 | SEQ ID NO: 08 |
| | QP14451 441 | QD1445 | SEQ ID NO: 13 | | QD1441 | SEQ ID NO: 09 |
| | QP14451 442 | QD1445 | SEQ ID NO: 13 | | QD 1442 | SEQ ID NO: 10 |

| | Protein number | Heavy chain plasmid number | Sequence number | | Light chain plasmid number | Sequence number |
|---|---|---|---|---|---|---|
| QP1992 00 humaniz ation design | QP14491 446 | QD1449 | SEQ ID NO: 17 | | QD1446 | SEQ ID NO: 14 |
| | QP14491 447 | QD1449 | SEQ ID NO: 17 | | QD1447 | SEQ ID NO: 15 |
| | QP14491 448 | QD1449 | SEQ ID NO: 17 | | QD1448 | SEQ ID NO: 16 |
| | QP14501 446 | QD1450 | SEQ ID NO: 18 | | QD1446 | SEQ ID NO: 14 |
| | QP14501 447 | QD1450 | SEQ ID NO: 18 | | QD1447 | SEQ ID NO: 15 |
| | QP14501 448 | QD1450 | SEQ ID NO: 18 | | QD1448 | SEQ ID NO: 16 |
| | QP14511 446 | QD1451 | SEQ ID NO: 19 | | QD1446 | SEQ ID NO: 14 |
| | QP14511 447 | QD1451 | SEQ ID NO: 19 | | QD1447 | SEQ ID NO: 15 |
| | QP14511 448 | QD1451 | SEQ ID NO: 19 | | QD1448 | SEQ ID NO: 16 |
| QP2012 02 humaniz ation design | QP14561 452 | QD1456 | SEQ ID NO: 24 | | QD1452 | SEQ ID NO: 20 |
| | QP14561 453 | QD1456 | SEQ ID NO: 24 | | QD1453 | SEQ ID NO: 21 |
| | QP14561 454 | QD1456 | SEQ ID NO: 24 | | QD1454 | SEQ ID NO: 22 |
| | QP14561 455 | QD1456 | SEQ ID NO: 24 | | QD1455 | SEQ ID NO: 23 |
| | QP14571 452 | QD1457 | SEQ ID NO: 25 | | QD1452 | SEQ ID NO: 20 |
| | QP14571 453 | QD1457 | SEQ ID NO: 25 | | QD1453 | SEQ ID NO: 21 |
| | QP14571 454 | QD1457 | SEQ ID NO: 25 | | QD1454 | SEQ ID NO: 22 |
| | QP14571 455 | QD1457 | SEQ ID NO: 25 | | QD1455 | SEQ ID NO: 23 |
| | QP14581 452 | QD1458 | SEQ ID NO: 26 | | QD1452 | SEQ ID NO: 20 |
| | QP14581 453 | QD1458 | SEQ ID NO: 26 | | QD1453 | SEQ ID NO: 21 |
| | QP14581 454 | QD1458 | SEQ ID NO: 26 | | QD1454 | SEQ ID NO: 22 |
| | QP14581 455 | QD1458 | SEQ ID NO: 26 | | QD1455 | SEQ ID NO: 23 |
| | QP14591 452 | QD1459 | SEQ ID NO: 27 | | QD1452 | SEQ ID NO: 20 |
| | QP14591 453 | QD1459 | SEQ ID NO: 27 | | QD1453 | SEQ ID NO: 21 |
| | QP14591 454 | QD1459 | SEQ ID NO: 27 | | QD1454 | SEQ ID NO: 22 |
| | QP14591 455 | QD1459 | SEQ ID NO: 27 | | QD1455 | SEQ ID NO: 23 |
| QP2072 08 humaniz ation design | Protein number | Heavy chain plasmid number | Sequence number | | Light chain plasmid number | Sequence number |
| | QP14631 460 | QD1463 | SEQ ID NO: 31 | | QD1460 | SEQ ID NO: 28 |
| | QP14631 461 | QD1463 | SEQ ID NO: 31 | | QD1461 | SEQ ID NO: 29 |
| | QP14631 462 | QD1463 | SEQ ID NO: 31 | | QD1462 | SEQ ID NO: 30 |
| | QP14641 460 | QD1464 | SEQ ID NO: 32 | | QD1460 | SEQ ID NO: 28 |
| | QP14641 461 | QD1464 | SEQ ID NO: 32 | | QD1461 | SEQ ID NO: 29 |
| | QP14641 462 | QD1464 | SEQ ID NO: 32 | | QD1462 | SEQ ID NO: 30 |
| | QP14651 460 | QD1465 | SEQ ID NO: 33 | | QD1460 | SEQ ID NO: 28 |
| | QP14651 461 | QD1465 | SEQ ID NO: 33 | | QD1461 | SEQ ID NO: 29 |
| | QP14651 462 | QD1465 | SEQ ID NO: 33 | | QD1462 | SEQ ID NO: 30 |
| | QP14661 460 | QD1466 | SEQ ID NO: 34 | | QD1460 | SEQ ID NO: 28 |
| | QP14661 461 | QD1466 | SEQ ID NO: 34 | | QD1461 | SEQ ID NO: 29 |
| | QP14661 462 | QD1466 | SEQ ID NO: 34 | | QD1462 | SEQ ID NO: 30 |

At the same time, clones were designed to express chimeric antibody before humanization and control antibody as shown in the following table; in this table, kappa light chain constant region CL is employed for all antibody light chains, and human IgG1 constant region is employed for the antibody heavy chains:

**Table 2: chimeric antibody before humanization and control antibody (IMAB362 is the control antibody corresponding to the protein number QP024025)**

| Hybridom a clone number | Protein number | Heavy chain plasmid number | Sequence number | | Light chain plasmid number | Sequence number |
|---|---|---|---|---|---|---|
| 175D10(I MAB362) | QP0240 25 | QD025 | SEQ ID NO: 36 | | QD024 | SEQ ID NO: 35 |
| 36A2 | QP1901 91 | QD191 | SEQ ID NO: 38 | | QD190 | SEQ ID NO: 37 |
| 40F1 | QP1921 93 | QD193 | SEQ ID NO: 40 | | QD192 | SEQ ID NO: 39 |
| 43C11 | QP 1992 00 | QD200 | SEQ ID NO: 42 | | QD199 | SEQ ID NO: 41 |
| 44C6 | QP2012 2 | QD202 | SEQ ID NO: 44 | | QD201 | SEQ ID NO: 43 |
| 51A3 | QP2072 08 | QD208 | SEQ ID NO: 46 | | QD207 | SEQ ID NO: 45 |
| phage screening | QP1115 1116 | QD1115 | SEQ ID NO: 48 | | QD1116 | SEQ ID NO: 47 |

### 2. Expression of humanized anti-Claudin18.2 antibodies

The culture density of 293E cells was maintained between 0.2-3×10⁶/ml, and a maintenance phase medium (GIBCO Freestyle 293 expression medium) was used for culture; the cells to be transfected were centrifuged one day before transfection, the medium was replaced, and the cell density was adjusted to 0.5-0.8×10⁶/ml. On the day of transfection, the density of 293E cells was 1-1.5 ×10⁶/ml. The plasmid and transfection reagent PEI were prepared, the amount of plasmid to be transfected was 100µg/100 ml cells, and the mass ratio of PEI to the plasmid as used was 2:1. The plasmid and PEI were mixed uniformly, then allowed to stand for 15 min (should not exceed 20 min). The mixture of the plasmid and PEI was slowly added into the 293E cells, and cultured in a shaker at 8% CO2, 120 rpm and 37°C; on the fifth day of transfection, the cell supernatant was collected after centrifuging in a horizontal centrifuge at 4700 rpm for 20 min.

### 3. Purification of humanized anti-Claudin18.2 antibodies

### Purification by protein A affinity chromatography

At least 3CV (actual volume 20 ml) of balance solution was allowed to pass through the column, so as to ensure that the pH and conductivity of the final solution flowing out of the instrument are consistent with the balance solution, and the flow rate was 1 ml/min; the culture supernatant after centrifugation was allowed to pass through the column, 40 ml of sample are was loaded, and the flow rate was 0.33 ml/min; at least 3CV (actual volume 20 ml) of balance solution was allowed to pass through the column, so as to ensure that the pH and conductivity of the final solution flowing out of the instrument are consistent with the balance solution, and the flow rate was 0.33 ml/min; the eluent was allowed to pass through the column, and the elution peaks (PAC-EP) began to be collected when the UV280 increased to 15 mAU; and collection stopped when the UV280 decreased to 15 mAU, and the flow rate was 1 ml/min. After the sample collection was completed, the PAC-EP was adjusted to neutral with a pH adjusting solution.

### 4. Binding activity of humanized Claudin18.2 antibodies (CHOS-CLDN18.2 cell-ELISA)

Detection reagents: skimmed milk powder (BD, 232100), PBS (Sangon, B548117-0500); HRP-anti human IgG(H+L) (jackson, 109-035-088); TMB (Luoyang Baiaotong Experimental Materials Center, C060201); Elisa plate (costa, 9018) 1×PBS buffer: 8.00 g ofNaCl, 0.20g ofKCl, 2.9g ofNa₂HPO₄•12H₂O, and 0.2g of KH₂PO₄ were weighed, and dissolved in 800mL of ddH₂O, the volume was adjusted to 1L after full dissolution, the pH was adjusted to 7.4, and the solution was sterilized at high temperature for use. Alternatively, commercial 10 ×, 20× PBS solutions were purchased and diluted to 1 ×PBS buffer for use. Blocking solution: 5g of milk powder was weighed into PBS; the blocking solution should be prepared immediately before use. Stop solution (1mol/L H₂SO₄): 109ml of 98% concentrated H₂SO₄ was slowly added dropwise into 2000mL of ddH₂O. 100µl/well of TMB was added for color development at 37°C for 10 min, and the plate was placed in a shaker (120 rpm);

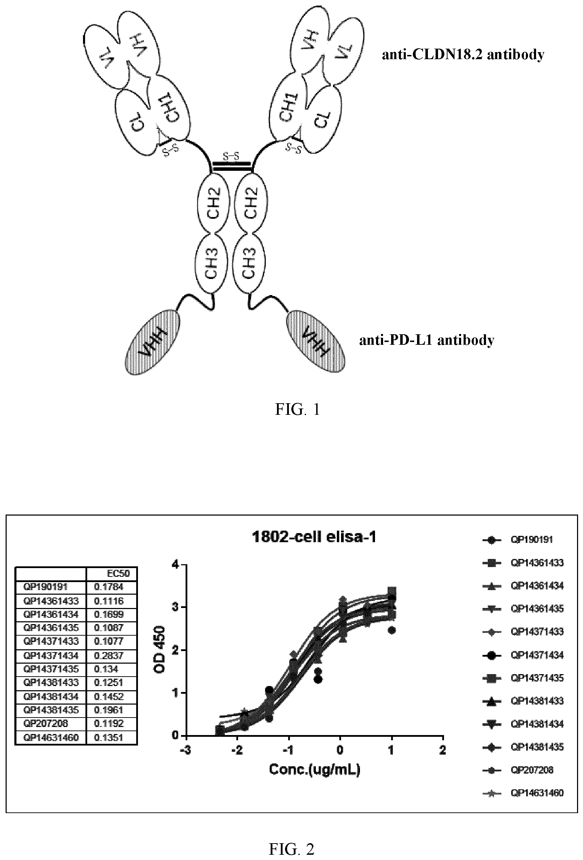

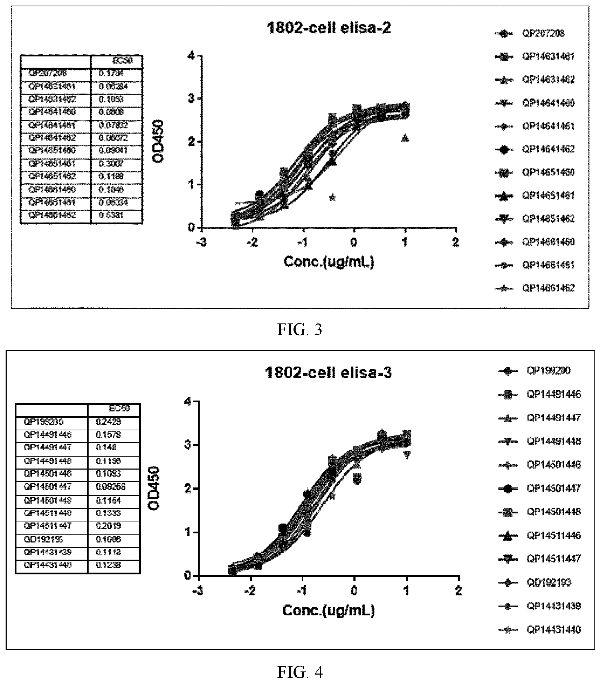

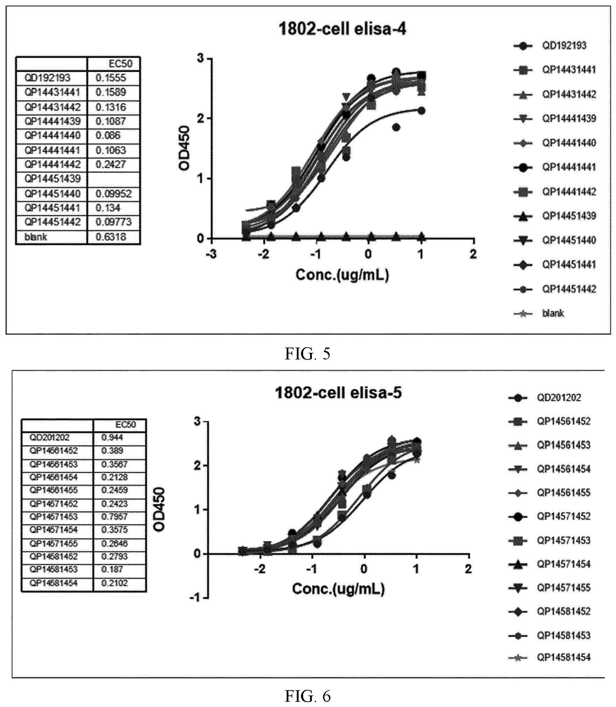

**Experimental steps:** cells CHOS-CLD18.2-16-2 were inoculated into an U-shaped plate at 2E5/well, then washing once with ice PBS, and centrifuging at 1200 rpm for 3 min. VCD: 1.21E6, actually 165 µl/well was plated; 200µL/well of blocking solution was added after completing cleaning and incubated on ice for 1 h. The blocking was completed followed by centrifuging at 1200 rpm for 3 min, discarding the supernatant, incubating CLD18.2 control antibody 14-1 and the sample, diluting according to 100 µg/ml in the dilution ratio of 1:2 for 12 gradients, and finally set a blank control; adding 100 µl/well according to 100 µg/mL, 33.33333333 µg/mL, 11.11111111 µg/mL, 3.703703704 µg/mL, 1.234567901 µg/mL, 0.411522634 µg/mL, 0.137174211 µg/mL, 0.045724737 µg/mL, 0.015241579 µg/mL, 0.005080526 µg/mL, 0.001694 µg/mL, 0µg/mL, fully mixing, incubating on ice for 2 h, and washing with ice PBS for 3 times; adding enzyme-labeled antibody: incubating HRP-anti human IgG(H+L) antibody, mixing thoroughly according to 100µl/well in the dilution ratio of 1:10000, then placing on ice for 1 h and washing with ice PBS for 3 times. Addition of substrate color-developing solution: substrate color-developing solution TMB was added according to the dosage of 100 µL/well, then placing in a shaker at 200rpm, and developing color at 35°C away from light for 10 min. Stop reaction: the color development was completed followed by rapidly adding stop solution according to the dosage of 100 µL/well to stop the reaction. Detection: after centrifugation at 3500rpm for 5 min, 120 µl of supernatant was transferred to an Elisa plate, and the OD value at A450 nm was measured on a microplate reader; the results were analyzed using graphpad prism software: as shown in FIGs. 2, 3, 4, 5 and 6.

The experimental results were shown in FIGs. 2, 3, 4, 5 and 6, which proved that all of the humanized Claudin 18.2 antibodies of the present disclosure bound to CHOS-Claudin 18.2.

### 5. Binding activity of humanized Claudin18.2 antibodies (CHOS-CLDN18.2 FACS)

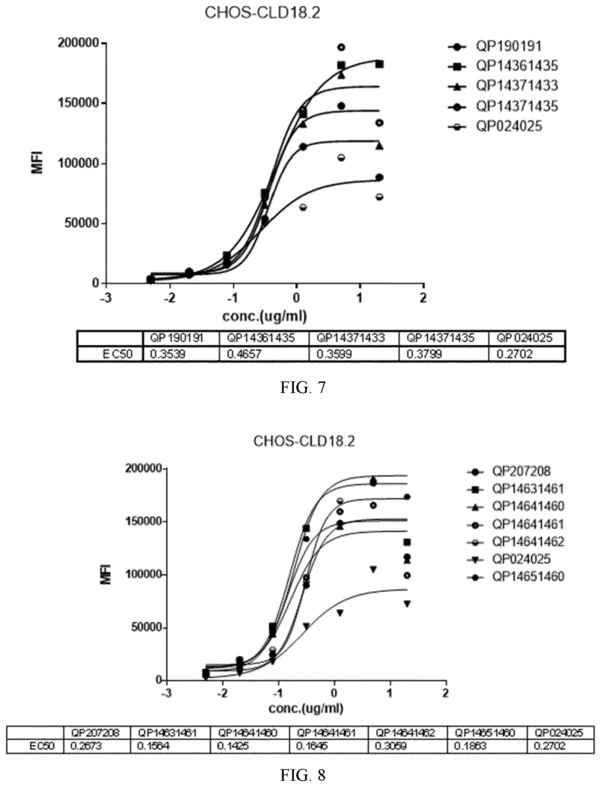

Cells CHOS and CHOS-CLDN18.2 were respectively collected in 2E5/well, centrifuged at 1000r for 5 min, and blocked with 200 µl/well of 3% BSA/PBS buffer at 4°C for 60 min. The initial concentration of the antibody was 20µg/ml, it was diluted at 1:4, and incubated at 4°C for 60 min. It was followed by washing with PBS for 2 times, incubating 50 µl/well of PE-anti-human FC (1:200), incubating at 4°C for 30 min, washing with PBS three times, and resuspending in PBS; the FACS results are as follows: Table 3, Table 4, Table 5, Table 6, Table 7 and FIG. 7, FIG. 8, FIG. 9, FIG. 10 and FIG. 11.

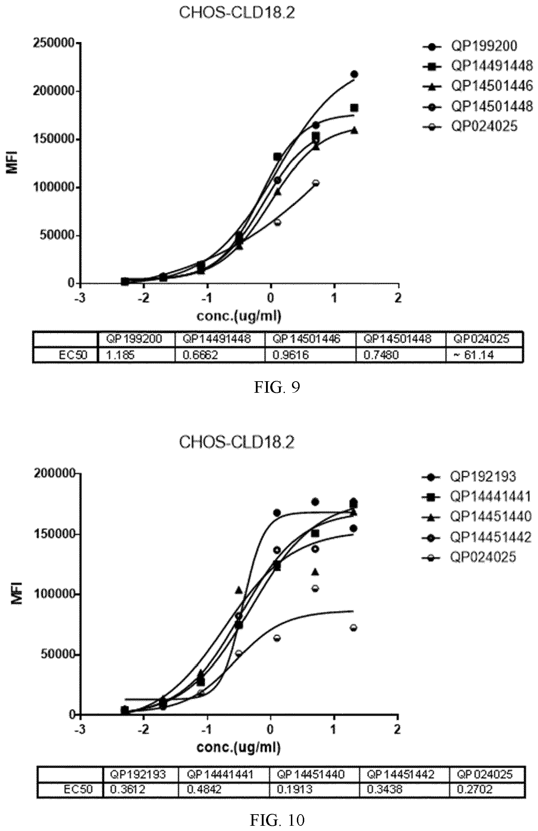

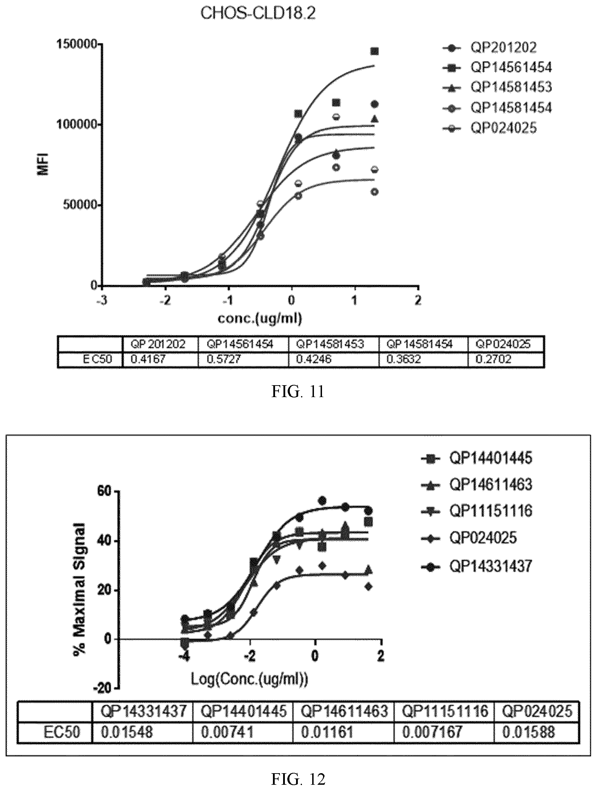

The EC50 and Max values in Table 3 and FIG.7 prove that the binding of humanized antibody QP14361435, QP14371433 and QP14371435 of mouse-derived chimeric antibody QP190191 to CHOS-CLDN18.2 cells is better than or equivalent to that of mouse-derived chimeric antibody QP190191; the EC50 and Max values in Table 7 and FIG. 11 prove that the binding of humanized antibody QP14561454, QP14581453 and QP14581454 of mouse-derived chimeric antibody QP201202 to CHOS-CLDN18.2 cells is equivalent to that of mouse-derived chimeric antibody QP201202; the EC50 and Max values in Table 6 and FIG. 10 prove that the binding of humanized antibody QP14451440, QP14441441 and QP14451442 of mouse-derived chimeric antibody QP192193 to CHOS-CLDN18.2 cells is equivalent to that of mouse-derived chimeric antibody QP192193; the EC50 and Max values in Table 5 and FIG. 9 prove that the binding of humanized antibody QP14491448, QP14501446, and QP14501448 of mouse-derived chimeric antibody QP199200 to CHOS-CLDN18.2 cells is equivalent to that of mouse-derived chimeric antibody QP199200; and the EC50 and Max values in Table 4 and FIG. 8 prove that the binding of humanized antibody QP14631461, QP14641460, QP14641461, QP14641462 and QP14651460 of mouse-derived chimeric antibody QP207208 to CHOS-CLDN18.2 cells is superior to or equivalent to that of mouse-derived chimeric antibody QP207208.

**Table 3: FACS determination of activities for the first group of humanized Claudin 18.2 antibodies**

| conc.(µg/ml) | QP190191 | QP14361435 | **QP14371433** | QP14371435 | QP024025 |
|---|---|---|---|---|---|
| 20 | 88700 | 183000 | 115000 | 134000 | 72300 |
| 5 | 148000 | 182000 | 174000 | 197000 | 105000 |
| 1.250 | 114000 | 141000 | 133000 | 145000 | 63700 |
| 0.313 | 54200 | 75900 | 65900 | 72000 | 51000 |
| 0.078 | 15700 | 23600 | 17700 | 18000 | 18200 |
| 0.020 | 8004 | 8525 | 10800 | 10200 | 6946 |
| 0.005 | 2840 | 3389 | 3726 | 4132 | 2829 |
| 0 | 729 | 713 | 754 | 732 | 732 |
| | | | | | |

| | QP190191 | QP14361435 | **QP14371433** | QP14371435 | QP024025 |
|---|---|---|---|---|---|
| EC50 | 0.3539 | 0.4657 | 0.3599 | 0.3799 | 0.2702 |
| max | 148000 | 182000 | 174000 | 197000 | 88650 |

**Table 4: FACS determination of activities for the second group of humanized Claudin 18.2 antibodies**

| conc. (µg/ ml) | QP207 208 | **QP14631 461** | QP14641 460 | QP14641 461 | QP14641 462 | QP14651 460 | QP024 025 |
|---|---|---|---|---|---|---|---|
| 20 | 117000 | 131000 | 114000 | 99500 | 117000 | 174000 | 72300 |
| 5 | 187000 | 227000 | 191000 | 166000 | 224000 | 189000 | 105000 |
| 1.25 0 | 149000 | 201000 | 146000 | 160000 | 170000 | 219000 | 63700 |
| 0.31 3 | 90200 | 144000 | 151000 | 97500 | 93600 | 134000 | 51000 |
| 0.07 8 | 24700 | 51400 | 44300 | 46300 | 29300 | 50300 | 18200 |
| 0.02 0 | 11500 | 19300 | 16200 | 19900 | 15800 | 19600 | 6946 |
| 0.00 5 | 5093 | 7786 | 6596 | 5978 | 8454 | 7186 | 2829 |
| 0 | 732 | 732 | 732 | 732 | 732 | 732 | 732 |
| | QP207 208 | **QP14631 461** | QP 14641 460 | QP 14641 461 | QP 14641 462 | QP14651 460 | QP024 025 |
| EC5 0 | 0.2673 | 0.1564 | 0.09356 | 0.1645 | 0.3059 | 0.1863 | 0.2702 |
| max | 187000 | 227000 | 191000 | 166000 | 224000 | 189000 | 88650 |

**Table 5: FACS determination of activities for the third group of humanized Claudin 18.2 antibodies**

| conc.(µg/ml) | QP199200 | **QP14491448** | QP14501446 | QP14501448 | QP024025 |
|---|---|---|---|---|---|
| 20 | 218000 | 183000 | 160000 | 117000 | 72300 |
| 5 | 165000 | 154000 | 143000 | 149000 | 105000 |
| 1.250 | 132000 | 132000 | 96200 | 108000 | 63700 |
| 0.313 | 50600 | 45100 | 39500 | 45600 | 51000 |
| 0.078 | 19900 | 19200 | 13900 | 14200 | 18200 |
| 0.020 | 7519 | 6321 | 6347 | 7455 | 6946 |
| 0.005 | 2764 | 2406 | 2330 | 2476 | 2829 |
| 0 | 732 | 732 | 732 | 732 | 732 |
| | | | | | |

| | QP199200 | **QP14491448** | QP14501446 | QP14501448 | QP024025 |
|---|---|---|---|---|---|
| EC50 | 1.185 | 0.6662 | 0.9616 | 0.4978 | 0.2702 |
| max | 165000 | 154000 | 143000 | 149000 | 88650 |

**Table 6: FACS determination of activities for the fourth group of humanized Claudin 18.2 antibodies**

| conc.(µg/ml) | QP192193 | QP14441441 | **QP14451440** | QP14451442 | QP024025 |
|---|---|---|---|---|---|
| 20 | 155000 | 175000 | 169000 | 177000 | 72300 |
| 5 | 177000 | 151000 | 139000 | 138000 | 105000 |
| 1.250 | 168000 | 125000 | 123000 | 137000 | 63700 |
| 0.313 | 74600 | 74700 | 104000 | 82300 | 51000 |
| 0.078 | 27800 | 27300 | 35400 | 29800 | 18200 |
| 0.020 | 11400 | 10100 | 13800 | 11100 | 6946 |
| 0.005 | 3794 | 4234 | 4932 | 4792 | 2829 |
| 0 | 732 | 732 | 732 | 732 | 732 |
| | | | | | |

| | QP192193 | QP14441441 | **QP14451440** | QP14451442 | QP024025 |
|---|---|---|---|---|---|
| EC50 | 0.3612 | 0.4842 | 0.2225 | 0.3438 | 0.2702 |
| max | 177000 | 175000 | 169000 | 177000 | 88650 |

**Table 7: FACS determination of activities for the fifth group of humanized Claudin 18.2 antibodies**

| conc.(µg/ml) | QP201202 | QP14561454 | **QP14581453** | QP14581454 | QP024025 |
|---|---|---|---|---|---|
| 20 | 113000 | 146000 | 104000 | 58600 | 72300 |
| 5 | 81100 | 114000 | 83000 | 73700 | 105000 |
| 1.250 | 92700 | 107000 | 91400 | 56100 | 63700 |
| 0.313 | 38000 | 44800 | 33300 | 30800 | 51000 |
| 0.078 | 12600 | 13400 | 13600 | 11500 | 18200 |
| 0.020 | 4772 | 6393 | 5917 | 4472 | 6946 |
| 0.005 | 2263 | 2472 | 2416 | 2368 | 2829 |
| 0 | 732 | 732 | 732 | 732 | 732 |
| | | | | | |

| | QP201202 | QP14561454 | **QP14581453** | QP14581454 | QP024025 |
|---|---|---|---|---|---|
| EC50 | 0.4167 | 0.5727 | 0.4246 | 0.3632 | 0.2702 |
| max | 113000 | 146000 | 104000 | 73700 | 88650 |

### 6. Specificity identification of humanized Claudin18.2 antibodies

A technique of fluorescence activated cell sorting (FACS) was used to identify the binding specificity of the humanized antibody of the present disclosure to Claudin18.2 but not to Claudin18.1.

2E5/well of cells CHOS, CHOS-CLDN18.2, CHOS-CLDN18.1 were collected respectively, centrifuged at 1000 r for 5 min, and blocked with 200 µl/well of 3% bovine serum albumin/PBS buffer at 4°C for 60 min. The initial concentration of the antibody was 20 µg/ml,it was diluted at 1:4, and incubated at 4°C for 60 min. It was followed by washing with PBS twice, incubating 50 µl/well of PE-anti-human FC (1:200), incubating at 4°C for 30 min, washing with PBS three times, and resuspending in PBS; the average fluorescence value of FACS readings is shown in Table 8; FACS results show that the humanized anti-Claudin 18.2 antibody of the present disclosure binds to CHOS-Claudin 18.2 cells, and it does not bind to CHOS- Claudin 18.1 and CHOS cells, which proves that humanized anti-Claudin 18.2 antibody specifically binds to Claudin 18.2 but does not bind to Claudin 18.1.

**Table 8: mean fluorescence values of FACS readings**

| MFI | CHOS (10 µg/ml) | CHOS-CLDN18.1 (10 µg/ml) | CHOS-CLDN18.2 (10 µg/ml) | CLDN18.2/CLDN18.1 |
|---|---|---|---|---|
| QP024025 | 1599 | 1397 | 145000 | 104 |
| QP190191 | 1245 | 1063 | 450000 | 423 |
| QP14361435 | 1122 | 916 | 504000 | 550 |
| QP14371433 | 1157 | 967 | 433000 | 448 |
| QP14371435 | 1167 | 1030 | 512000 | 497 |
| QP207208 | 3115 | 1317 | 627000 | 476 |
| QP14631461 | 1201 | 938 | 657000 | 700 |
| QP14641460 | 1622 | 1044 | 604000 | 579 |
| QP14641461 | 1165 | 1017 | 721000 | 709 |
| QP14641462 | 1185 | 1056 | 758000 | 718 |
| QP14651460 | 1106 | 998 | 595000 | 596 |
| QP199200 | 1223 | 918 | 514000 | 560 |
| QP14491448 | 1159 | 994 | 484000 | 487 |
| QP14501446 | 1375 | 990 | 505000 | 510 |
| QP14501448 | 1369 | 1078 | 540000 | 501 |
| QP192193 | 1131 | 1027 | 644000 | 627 |
| QP14441441 | 1347 | 1139 | 693000 | 608 |
| QP14451440 | 1526 | 1319 | 620000 | 470 |
| QP14451442 | 1611 | 1411 | 653000 | 463 |
| QP201202 | 1168 | 1152 | 439000 | 381 |
| QP14561454 | 1067 | 1110 | 477000 | 430 |
| QP14581453 | 1114 | 953 | 554000 | 581 |
| QP14581454 | 1097 | 992 | 502000 | 506 |

### 7. ADCC (antibody-dependent cell-mediated cytotoxicity) of humanized anti-Claudin18.2 antibodies

**Preparation of target cells (HEK293-CLDN18.2):** HEK293-CLDN18.2 were digested with trypsin at 1000 rpm for 5 min. It was followed by replacing with fresh culture medium, plating on a 96-well plate in 20000 cells/well, and incubating at 37°C in 5% CO2 overnight. **Preparation of antibody:** antibody (80 µg/ml-0.000512 µg/ml, 0 µg/ml) were diluted to 10 concentrations with a culture medium in 1:5 gradient. The culture medium in the 96-well plate was suck out, and 70µl/well of the above diluted antibodies of each concentration were added, and duplicate wells were set for each concentration. **Preparation of PBMC:** the PBMCs resuscitated on the first day were centrifuged, suspended in the culture medium, and counted. 70U1/well of cells according to PBMC: target cell=50:1 were added into the above well plate, and incubated at 37°C for 4 hours. To Max lysis well, 15µl lysis buffer (1%Triton-X100) was added, and incubated at 37 degrees for 10 min.

**Preparation of LDH reagent:** 96-well plate was centrifuged at 200 g for 5 minutes, and 100 µl/well of supernatant was transferred to a new transparent 96-well plate. An LDH Cytotoxicity Assay Kit (cayman, 10008882-480well) was taken out to prepare a reaction solution, 100 µl/well was added, and gently shaken at 37 degrees for 30 minutes. Absorption readings at 490 nm were obtained, and the data were analyzed according to the formula Con(µg/ml)%Maximal signal=(Test-Control)/(Max-Control)-Con(0µg/ml)%Maximal signal.

The experimental results are shown in FIG. 12, table 9 and table 10, which show that all of the humanized anti-Claudin 18.2 antibodies QP14331437, QP14401445, and QP14611463, the whole human antibody QP11151116 and the control antibody IMAB362 (QP024025) can kill HEK293-Claudin 18.2 cells highly expressing Claudin 18.2 via concentration-dependent PBMC-mediated killing.

**Table 9: Readings at 490nm**

| conc.(µg/ ml) | 40 | 8 | 1.6 | 0.3 2 | 0.0 64 | 0.01 28 | 0.002 56 | 0.000 51 | 0.00 01 | 0 | MA X |
|---|---|---|---|---|---|---|---|---|---|---|---|
| QP14331 437 | 1.0 12 | 1.0 05 | 1.0 12 | 0.9 56 | 0.8 56 | 0.75 2 | 0.596 | 0.561 | 0.55 | 0.5 6 | 1.2 1 |
| QP14401 445 | 0.9 8 | 0.9 19 | 0.8 56 | 0.9 07 | 0.8 61 | 0.72 6 | 0.573 | 0.557 | 0.46 3 | 0.5 27 | 1.1 7 |
| QP14611 463 | 0.8 44 | 0.9 47 | 0.9 03 | 0.9 08 | 0.8 35 | 0.67 8 | 0.567 | 0.516 | 0.51 3 | 0.5 22 | 1.1 44 |
| QP11151 116 | 1.1 76 | 0.9 27 | 0.8 85 | 0.8 63 | 0.7 71 | 0.75 2 | 0.58 | 0.521 | 0.52 1 | 0.5 08 | 1.3 21 |
| QP02402 5 | 0.7 94 | 0.7 9 | 0.7 91 | 0.7 77 | 0.6 75 | 0.56 1 | 0.487 | 0.479 | 0.44 8 | 0.4 65 | 1.1 41 |
| Target+A b | 0.4 88 | 0.4 18 | 0.3 58 | 0.3 61 | 0.2 72 | 0.25 3 | 0.274 | 0.261 | 0.27 4 | 0.2 79 | |

**Table 10: Killing percentage**

| conc.(µg/ml ) | QP1433143 7 | QP1440144 5 | QP1461146 3 | QP1115111 6 | QP02402 5 |
|---|---|---|---|---|---|
| 40 | 52.32% | 47.80% | 28.62% | 75.45% | 21.57% |
| 8 | 53.76% | 42.72% | 46.32% | 43.75% | 26.16% |
| 1.6 | 56.36% | 37.77% | 43.37% | 41.22% | 30.02% |
| 0.32 | 49.58% | 43.72% | 43.84% | 38.46% | 28.17% |
| 0.064 | 41.55% | 42.09% | 39.27% | 32.36% | 21.98% |
| 0.0128 | 31.27% | 28.52% | 23.43% | 31.27% | 11.04% |
| 0.00256 | 13.30% | 10.80% | 10.15% | 11.56% | 1.49% |
| 0.000512 | 10.46% | 10.03% | 5.65% | 6.19% | 1.70% |
| 0.0001024 | 8.31% | -1.11% | 4.30% | 5.17% | -2.74% |
| 0 | 9.02% | 5.43% | 4.88% | 3.36% | -1.33% |
| EC50 | 0.0162 | 0.0082 | 0.01148 | 0.00716 | 0.01562 |

### 8. CDC (complement-dependent cytotoxicity) of humanized anti-Claudin18.2 antibodies

**Reagents:** cell: HEK293-hCLDN18.2-H11, complement: normal human serum complement (Quidel, A113), antibodies: QP024025 (IMAB362), QP14631461 from synthesis in this example.

### Experimental steps:

Plating of cell/culture medium: a background control group (culture media background) was set, that is, only culture medium 40 µl/well was added; and a maximal release group (maximum LDH release) was set, that is, after adding 40 µl of target cells, 12 µl lysis solution was added 45 min before the detection. A volume correction group (volume correction) was set, that is, culture medium 40 µl/well was added; a LDH positive control group was set, that is, 1 µl positive control was vortexed and then diluted by 5000 times (5mL) with a diluent PBS+1%BSA. It can be diluted with a gradient of 10 times. An experimental group was set, that is, 40µl/well of target cells were added. Addition of complement+antibody mix: 40 µl/well of 20% complement (diluted with cell culture medium) was added for both the control group and the experimental group. Cells were cultured at 37°C in 5% CO₂ for 1 h and 15 min, then 12 µl/well of lysis solution was added to the maximum release group and the volume correction group. Culture was continued at 37°C for 45 min, then a LDH detection kit was used for detection, and the absorbance readings at 490 nm were obtained, in which the readings should be completed within 1 h after adding the stop solution. Calculation: The background control group was subtracted from the experimental group and the volume correction group was subtracted from the maximum release group according to the formula as follows: Percent cytotoxicity=100*OD490(experimental LDH release)/OD490(maximum LDH release).

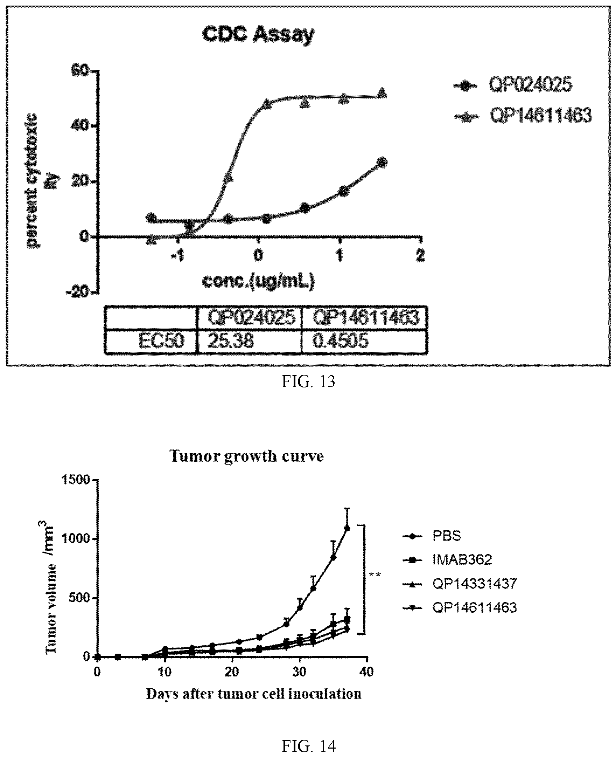

The CDC results are shown in FIG. 13; the results show that both the humanized anti-Claudin 18.2 antibody QP14611463 and the control antibody IMAB362 (QP024025) can kill HEK293-Claudin 18.2 cells highly expressing Claudin18.2 via concentration-dependent complement-mediated killing.

### 9. In vivo pharmacodynamics of humanized anti-Claudin18.2 antibodies in immunodeficient mice CB.17-SCID model HEK293-hClaudin18.2

**Experimental method:** HEK293-hClaudin18.2 cells which stably expressed human claudin18.2(hClaudin18.2) in logarithmic growth phase were collected, the cells were adjusted to concentration of 5×10⁷/mL with PBS buffer, and 0.1 ml (1:1 Matrigel) of cell suspension was inoculated subcutaneously in the right flank of CB-17 SCID mice. The mice after inoculation were observed and the tumor growth was monitored; the weight of mice was divided into groups on the day of inoculation and observed after administration.

Experimental results: as shown in FIG. 14 and Table 11.

**Table 11: Tumor Volume**

| Group | Dosage (mg/kg) | Tumor volume (mean±standard error, mm³) | Tumor growth inhibition rate (%) | P value |
|---|---|---|---|---|
| | | | | vs PBS |
| Vehicle control | -- | 1091.34±169.24 | -- | -- |
| group (PBS) | | | | |
| IMAB362 | 10 | 324.19±87.39 | 70.29% | 0.0006** |
| QP14331437 | 10 | 260.65±65.90 | 76.12% | 0.0001** |
| QP14611463 | 10 | 225.01±68.44 | 79.38% | 0.0001** |

The molecules to be detected are humanized claudin18.2 antibodies QP14331437 and QP14611463, and the positive control antibody is IMAB362 (QP024025). Dosing scheme: 10mpk×10, q2d, *i.v*. administered. On the 37th day after administration, the mean tumor volume of PBS group (negative control group) reached 1091.34mm³, the mean tumor volume of QP14331437 group was 260.65mm³ and TGI=76.12%, the mean tumor volume of QP14611463 group was 225.01mm³ and TGI=79.38%, and the mean tumor volume of IMAB362 group was 324.19mm³ and TGI=70.29%; there are statistically extremely significant differences in tumor volume between the three groups and PBS group (T test, p<0.01).

The experiment illustrates that our humanized anti-claudin18.2 antibody shows a trend of anti-tumor ability superior to that of the control antibody IMAB362 in HEK293-CLDN18.2 model.

### Example 2: Humanization of anti-PD-L1 single-domain antibodies

By means of aligning the germline gene database of the heavy and light chain variable regions of IMGT human antibody using MOE software, the germline genes of heavy and light chain variable regions having high homology with QP1162 and QP1166 were selected respectively as templates, and the CDRs of a single-domain antibody were grafted into the corresponding human templates IGHV3-23 germline and J-region IGHJ4*01, and a variable region sequence in an order of FR1-CDR1-FR2-CDR2-FR3-CDR3-FR4 was formed. Then, some important amino acid residues are selected for reverse mutation combinations. Among them, the amino acid residues were identified and annotated by Kabat numbering system.

### 1. Molecular cloning of humanized anti-PD-L1 single-domain antibodies

Primer PCR was designed to construct VH gene fragments of humanized antibody, and then homologous recombination was carried out with expression vector pQD having signal peptide and constant region gene (FC) fragments to construct an antibody full-length expression vector VH-FC-pQD.

Online software DNAWorks (v3.2.2)(http://helixweb.nih.gov/dnaworks/) was used to design a number of primers to synthesize VH/VK containing the gene fragment needed for recombination: 5'-30bp signal peptide +VH+30bp FC-3'. According to the operating instructions of Primer STAR GXL DNA polymerase of TaKaRa Company, VH/VK containing gene fragment needed for recombination was obtained by two-step PCR amplification using the primers designed above. Construction and enzyme digestion of expression vector pQD having signal peptide and constant region gene (FC) fragment: restriction enzyme such as BsmBI, as well as the different characteristics of the recognition sequence from that of the digestion sites were used to design and construct the expression vector pQD having signal peptide and constant region gene (FC) fragment. The vector was digested using BsmBI enzyme, and the gel was cut and recovered for later use. An expression vector VH-FC-pQD was recombinantly constructed. The VH containing the gene fragment required for recombination and expression vector pQD (having signal peptide and constant region gene (FC) fragment) recovered by BsmBI enzyme digestion were added into DH5H competent cells at a ratio of 3:1, then subjected to ice bath at 0°C for 30 min, heat shock at 42°C for 90s, followed by adding 5 times ofLB medium, incubating at 37°C for 45 min, coating LB-Amp plate, culturing overnight at 37°C, and picking single clones for sequencing to obtain individual clones of interest.

The humanization design for the sequences of the light and heavy chain variable regions of individual clones and the protein expression numbers are shown below; in this table, the C terminal of the antibody is fused to human IgG1-FC constant region:

**Table 12: humanization design of QP1162 and P 1166**

| | Protein number | Plasmid number | Sequence number |
|---|---|---|---|
| Humanization design of QP1162 | QP320 | QD320 | SEQ ID NO: 49 |
| | QP321 | QD321 | SEQ ID NO: 50 |
| | QP322 | QD322 | SEQ ID NO: 51 |

| | Protein number | Plasmid number | Sequence number |
|---|---|---|---|
| Humanization design of QP1166 | QP323 | QD323 | SEQ ID NO: 52 |
| | QP324 | QD324 | SEQ ID NO: 53 |
| | QP325 | QD325 | SEQ ID NO: 54 |

| | | | |
|---|---|---|---|
| * The humanized VHH has the same length as the original single-domain antibody of camel, both being 126 aa. | | | |

At the same time, clones were designed to express chimeric antibody before humanization and control antibody as shown in the following table:

**Table 13: chimeric antibody before humanization and control antibody**

| | Protein number | Plasmid number | Sequence number |
|---|---|---|---|
| QP1162 | QP1162 | QD1162 | SEQ ID NO: 55 |
| QP1166 | QP1166 | QD1166 | SEQ ID NO: 56 |
| 3280A (atezolimab) | QP11801181 | QD1181 | SEQ ID NO: 57 |
| | | QD1180 | SEQ ID NO: 58 |

### 2. Expression of protein of humanized anti-PD-L1 single-domain antibodies

The culture density of 293E cells was maintained at 0.2-3×10⁶/ml, and a maintenance phase medium (GIBCO Freestyle 293 expression medium) was used for culture; the cells to be transfected were centrifuged one day before transfection, the medium was replaced, and the cell density was adjusted to 0.5-0.8×10⁶/ml. On the day of transfection, the density of 293E cells was 1-1.5×10⁶/ml. The plasmid and transfection reagent PEI were prepared, the amount of plasmid to be transfected is 100µg/100ml cells, and the mass ratio of PEI to plasmid used is 2:1. The plasmid and PEI were mixed uniformly, then standing for 15 min (should not exceed 20 min). The mixture of plasmid and PEI was slowly added into 293E cells, and cultured in a shaker at 8% CO2, 120rpm and 37°C; on the fifth day of transfection, the cell supernatant was collected after centrifuging in a horizontal centrifuge at 4700rpm for 20 min.

### 3. Purification of protein of humanized anti-PD-L1 single-domain antibodies Purification by protein A affinity chromatography

At least 3CV (actual volume 20 ml) of balance solution was allowed to pass through the column, so as to ensure that the pH and conductivity of the final solution flowing out of the instrument are consistent with the balance solution, and the flow rate was 1 ml/min; the culture supernatant after centrifugation was allowed to pass through the column, 40 ml of sample are was loaded, and the flow rate was 0.33 ml/min; at least 3CV (actual volume 20 ml) of balance solution was allowed to pass through the column, so as to ensure that the pH and conductivity of the final solution flowing out of the instrument are consistent with the balance solution, and the flow rate was 0.33 ml/min; the eluent was allowed to pass through the column, and the elution peaks (PAC-EP) began to be collected when the UV280 increased to 15 mAU; and collection stopped when the UV280 decreased to 15 mAU, and the flow rate was 1 ml/min. After the sample collection was completed, the PAC-EP was adjusted to neutral with a pH adjusting solution.

### 4. Binding activity of humanized anti-PD-L1 single domain antibodies (Binding-ELISA)

50µl/well of antibodies QP1162/QP320/QP321/QP322/QP1166/QP323/QP324/ QP325 0.75µg/ml, and QP11801181 1.5 µg/ml were coated overnight at 4°C. P BS 3 times. Blocking: 3% BSA 250µl/well, placed at RT for 1 h. 2 µg/ml Bio tin QP004.3(biotin-PDL1-FC) were incubated respectively with different concent rations in 1:4 dilution at RT for 1h. PBST 3 times, PBS 3 times. Incubation o f secondary antibody: HRP-strepavidin (1:5000) 50 µl/well, PBST 6 times, PBS 3 times. Color development: TMB 100µl/well, color development was allowed for 10 min. 2M H₂SO₄ 50µl/well was added for stop reaction.

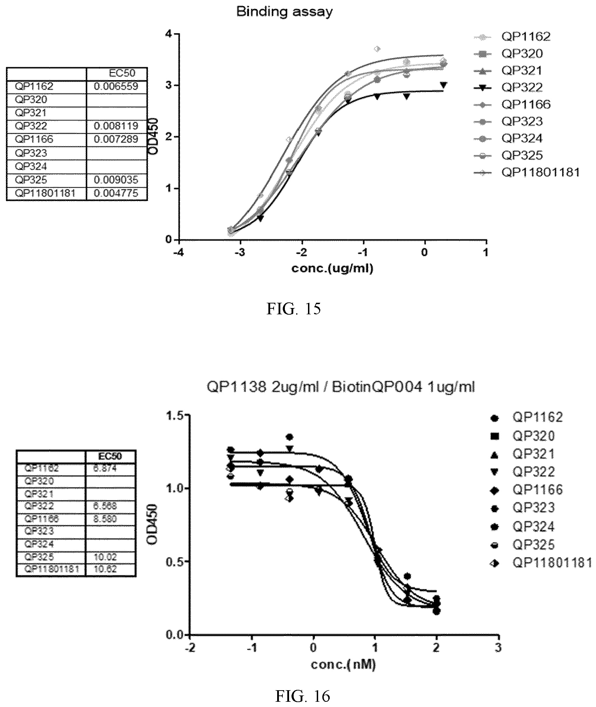

As shown in FIG. 15 and table 14, both humanized antibody QP322 of nanobody QP1162 and humanized antibody QP325 of nanobody QP1166 of the present disclosure can bind PD-L1 protein, which are equivalent to nanobody before humanization.

**Table 14: Results of activity identification (binding-ELISA) of humanized anti-PD-L1 single-domain antibodies**

| | QP11 62 | 1162-V1 | 1162-V2 | 1162-V3 | QP11 66 | 1166-V1 | 1166-V2 | 1166-V3 | QP11801 181 |
|---|---|---|---|---|---|---|---|---|---|
| Conc . (µg/ ml ) | QP11 62 | QP32 0 | QP32 1 | QP32 2 | QP11 66 | QP32 3 | QP32 4 | QP32 5 | |
| 2.000 0 | 3.436 6 | 0.229 7 | 0.225 1 | 3.004 2 | 3.414 3 | 2.856 4 | 0.413 0 | 3.421 6 | 3.4911 |
| 0.500 0 | 3.448 2 | 0.141 9 | 0.137 8 | 2.786 1 | 3.280 5 | 1.877 3 | 0.201 2 | 3.209 0 | 3.4778 |
| 0.166 7 | 3.259 7 | 0.095 7 | 0.083 6 | 2.781 1 | 3.134 3 | 0.789 3 | 0.128 5 | 3.105 9 | 3.7196 |
| 0.055 6 | 2.835 5 | 0.084 3 | 0.075 6 | 2.700 8 | 3.232 7 | 0.314 1 | 0.088 2 | 2.782 8 | 3.2350 |
| 0.018 5 | 2.501 6 | 0.079 2 | 0.096 0 | 2.071 5 | 2.566 0 | 0.181 0 | 0.107 0 | 2.124 6 | 2.5747 |
| 0.006 2 | 1.539 6 | 0.107 6 | 0.120 1 | 1.273 5 | 1.546 4 | 0.166 3 | 0.150 2 | 1.318 9 | 1.9496 |
| 0.002 1 | 0.523 2 | 0.114 0 | 0.156 1 | 0.410 7 | 0.560 5 | 0.257 7 | 0.141 4 | 0.597 8 | 0.8702 |
| 0.000 7 | 0.121 4 | 0.124 8 | 0.156 2 | 0.175 6 | 0.216 9 | 0.181 0 | 0.191 7 | 0.181 7 | 0.1986 |

### 5. Activity identification (Blocking-ELISA) of humanized anti-PD-L1 single-domain antibodies

6. To coat 50µl/well of protein QP1138 (PD1-FC) 2µg/ml overnight at 4°C. PBS 3 times. Blocking: 3% BSA 250µl/well, placed at RT for 1 h. 2 µg/ml Biotin QP004.3(biotin-PDL1-FC) and different concentrations of QP1120 15 µg/ml, QP11801181 30 µg/ml were prepared respectively, diluted at 1:3, uniformly mixed with equal volume, and placed at RT for 1 h. PBST 3 times, PBS 3 times. Incubation of secondary antibody: HRP-strepavidin (1:5000) 50 µl/well, PBST 6 times, PBS 3 times. Color development: TMB 100 µl/well, color development was allowed for 10 min. 2M H₂SO₄ 50 µl/well was added for stop reaction.

The experimental results are shown in FIG. 16 and table 15; humanized antibody QP322 derived from nanobody QP1162 and humanized antibody QP325 derived from nanobody QP1166 of the present disclosure can block the binding of PD-L1 to PD-1 protein, which is equivalent to the nanobody before humanization.

**Table 15: Activity identification (Blocking-ELISA) of humanized anti-PD-L1 single-domain antibodies**

| | QP11 62 | 1162-V1 | 1162-V2 | 1162-V3 | QP11 66 | 1166-V1 | 1166-V2 | 1166-V3 | QP1180 1181 |
|---|---|---|---|---|---|---|---|---|---|
| conc.(n M) | QP11 62 | QP32 0 | QP32 1 | QP32 2 | QP11 66 | QP32 3 | QP32 4 | QP32 5 | |
| 100.00 00 | 0.249 2 | 0.793 5 | 0.976 7 | 0.202 7 | 0.167 0 | 3.638 1 | 0.661 2 | 0.156 2 | 0.2121 |
| 33.333 3 | 0.400 6 | 1.017 1 | 1.072 0 | 0.279 6 | 0.238 7 | 2.110 3 | 0.736 6 | 0.238 5 | 0.3223 |
| 11.111 1 | 0.523 9 | 1.121 0 | 0.996 6 | 0.483 6 | 0.508 4 | 1.597 1 | 0.752 8 | 0.512 7 | 0.5794 |
| 3.7037 | 1.067 4 | 1.112 0 | 1.014 8 | 0.914 0 | 1.059 3 | 1.218 4 | 0.895 4 | 1.028 4 | 0.8983 |
| 1.2346 | 1.131 0 | 1.077 1 | 1.097 3 | 0.969 1 | 1.131 6 | 1.081 0 | 0.878 6 | 0.994 6 | 0.9870 |
| 0.4115 | 1.350 1 | 1.467 9 | 1.439 6 | 1.268 4 | 1.061 1 | 1.104 8 | 0.901 5 | 0.975 2 | 0.9298 |
| 0.1372 | 1.179 7 | 1.295 1 | 1.140 8 | 1.105 7 | 1.242 6 | 1.146 4 | 1.061 5 | 1.020 3 | 1.0141 |
| 0.0457 | 1.264 0 | 1.214 6 | 1.370 2 | 1.208 2 | 1.156 2 | 1.343 4 | 1.288 7 | 1.081 7 | 1.1329 |

### 7. Affinity identification of humanized anti-PD-L1 single-domain antibodies by SPR

Surface plasmon resonance (SPR) was used to detect affinity.

The affinity of the molecules to be detected to protein PD-L1 and cynoPD-L1 was determined by Biacore T200 (GE).

The antigen information is as follows:

**Table 16: Protein Number**

| Protein number | Protein description | Article number |
|---|---|---|
| QPP09.1 | PD-L1 Protein, Human, Recombinant (His Tag) | SinoBiologic, 10084-H08H |
| QPP10.1 | PD-L1 Protein, Cynomolgus, Recombinant (His Tag) | SinoBiologic, 90251-C08H |

**Table 17: results of affinity by SPR**

| | | | | | | | | | |
|---|---|---|---|---|---|---|---|---|---|
| kinetic s model | channe l | capture 1 solution | analyte 1 solution | kinetics Chi²(RU² ) | Ka(1/Ms ) | kd(1/s) | KD(M) | Rmax(RU ) | tc |
| 1:1 bindin g | 1 | QP1122 | QPP09. 1 | 5.32E+0 0 | 1.19E+0 7 | 3.89E-02 | 3.26E-09 | 107.3 | 6.00E+0 7 |
| 1:1 bindin g | 1 | | QPP10. 1 | 1.17E+0 0 | 4.64E+0 6 | 3.04E-02 | 6.55E-09 | 123.5 | 5.71E+0 7 |
| 1:1 bindin g | 2 | QP 1126 | QPP09. 1 | 3.99E+0 0 | 1.83E+0 9 | 5.69E+0 0 | 3.10E-09 | 46.5 | 1.66E+0 7 |
| 1:1 bindin g | 2 | | QPP10. 1 | 8.59E-01 | 9.44E+0 8 | 7.43E+0 0 | 7.87E-09 | 49.5 | 1.52E+0 7 |
| 1:1 bindin g | 3 | QP1162 | QPP09. 1 | 7.35E-01 | 1.37E+0 6 | 3.79E-03 | 2.77E-09 | 119.5 | 1.19E+0 8 |
| 1:1 bindin g | 3 | | QPP10. 1 | 4.81E-01 | 7.15E+0 5 | 4.01E-03 | 5.60E-09 | 130.2 | 1.64E+0 8 |
| 1:1 bindin g | 4 | QP322 | QPP09. 1 | 1.40E+0 0 | 1.31E+0 6 | 3.11E-03 | 2.37E-09 | 110.4 | 1.15E+0 8 |
| 1:1 bindin g | 4 | | QPP10. 1 | 3.91E-01 | 6.57E+0 5 | 3.54E-03 | 5.39E-09 | 123.1 | 1.41E+0 8 |
| 1:1 bindin g | 5 | QP1166 | QPP09. 1 | 6.52E-01 | 9.68E+0 5 | 2.55E-03 | 2.63E-09 | 107 | 1.29E+0 8 |
| 1:1 bindin g | 5 | | QPP10. 1 | 1.68E-01 | 4.75E+0 5 | 2.66E-03 | 5.60E-09 | 122.4 | 2.11E+0 8 |
| 1:1 bindin g | 6 | QP325 | QPP09. 1 | 3.19E-01 | 3.75E+0 5 | 4.00E-03 | 1.07E-08 | 115.2 | 1.27E+0 8 |
| 1:1 bindin g | 6 | | QPP10. 1 | 3.51E-02 | 2.12E+0 5 | 4.16E-03 | 1.96E-08 | 127.3 | 1.25E+0 9 |
| 1:1 bindin g | 7 | QP1180118 1 | QPP09. 1 | 1.23E-01 | 2.56E+0 5 | 2.19E-04 | 8.57E-10 | 44.5 | 1.31E+0 8 |
| 1:1 bindin g | 7 | | QPP10. 1 | 1.80E-01 | 3.91E+0 8 | 3.75E+0 0 | 9.60E-09 | 11.7 | 1.04E+0 6 |

The experimental results illustrated that the result of affinity via SPR showed that both humanized anti-PD-L1 antibodies QP322 and QP325 bind human PD-L1 protein and monkey PD-L1 protein. Among them, humanized nanobody QP322 and camel nanobody QP1162 have similar binding affinity to human and monkey PD-L1 protein, and the humanization thereof is successful.

### Example 3: anti-CLDN18.2/anti-PD-L1 bispecific antibodies

In this example, the humanized nanobody QP322 and the heavy chain of anti-CLDN18.2 form a fusion protein via linker peptide (G₄S)₄, which was used to construct anti-CLDN18.2/anti-PD-L1 bispecific antibody.

The form of the designed anti-CLDN18.2/anti-PD-L1 bispecific antibody molecule is shown in FIG. 1.

### 1. Construction of anti-CLDN18.2/anti-PD-L1 bispecific antibodies

Primer PCR was designed to construct VH gene fragments of humanized antibody, and then homologous recombination was carried out with expression vector pQD (having signal peptide and constant region gene fragment) to construct an antibody full-length expression vector pQD.

The sequences of anti-CLDN18.2/anti-PD-L1 bispecific antibodies and protein expression numbers are as follows:

**Table 18: sequence of anti-CLDN18.2/anti-PD-L1 bispecific antibody and protein expression number**

| Protein number | Plasmid number | Sequence number | Description |
|---|---|---|---|
| QP369143 3 | QD1433 | SEQ ID NO: 59 | pQD-antiCLD18.2(QD1433)VL-CL |
| | QD369 | SEQ ID NO: 60 | pQD-antiCLD18.2(QD1437)VH-CH1-FC-antiPDL1(QD322) |
| QP370144 0 | QD 1440 | SEQ ID NO: 61 | pQD-antiCLD18.2(QD1440)VL-CL |
| | QD370 | SEQ ID NO: 62 | pQD-antiCLD18.2(QD1445)VH-CH1-FC-antiPDL1(QD322) |
| QP3711461 | QD 1461 | SEQ ID NO: 63 | pQD-antiCLD18.2(QD1461)VL-CL |
| | QD371 | SEQ ID NO: 64 | pQD-antiCLD18.2(QD1463)VH-CH1-FC-antiPDL1(QD322) |
| QP3721116 | QD1116 | SEQ ID NO: 65 | pQD-antiCLD18.2(QD1116)VL-CL |
| | QD372 | SEQ ID NO: 66 | pQD-antiCLD18.2(QD1115)VH-CH1-FC-antiPDL1(QD322) |

### 2. Expression of anti-CLDN18.2/anti-PD-L1 bispecific antibodies

The culture density of 293E cells was maintained at 0.2-3×106/ml, and a maintenance phase medium (GIBCO Freestyle 293 expression medium) was used for culture; the cells to be transfected were centrifuged one day before transfection, the medium was replaced, and the cell density was adjusted to 0.5-0.8×106/ml. On the day of transfection, the density of 293E cells was 1-1.5×106/ml. The plasmid and transfection reagent PEI were prepared, the amount of plasmid to be transfected is 100ug/100ml cells, and the mass ratio of PEI to plasmid used is 2: 1. The plasmid and PEI were mixed uniformly, then standing for 15 min (should not exceed 20 min). The mixture of plasmid and PEI was slowly added into 293E cells, and cultured in a shaker at 8% CO2, 120rpm and 37°C; on the fifth day of transfection, the cell supernatant was collected after centrifuging in a horizontal centrifuge at 4700rpm for 20 min.

### 3. Expression and purification of anti-CLDN18.2/anti-PD-L1 bispecific antibodies

### Purification by protein A affinity chromatography

At least 3CV (actual volume 20 ml) of balance solution was allowed to pass through the column, so as to ensure that the pH and conductivity of the final solution flowing out of the instrument are consistent with the balance solution, and the flow rate was 1 ml/min; the culture supernatant after centrifugation was allowed to pass through the column, 40 ml of sample are was loaded, and the flow rate was 0.33 ml/min; at least 3CV (actual volume 20 ml) of balance solution was allowed to pass through the column, so as to ensure that the pH and conductivity of the final solution flowing out of the instrument are consistent with the balance solution, and the flow rate was 0.33 ml/min; the eluent was allowed to pass through the column, and the elution peaks (PAC-EP) began to be collected when the UV280 increased to 15 mAU; and collection stopped when the UV280 decreased to 15 mAU, and the flow rate was 1 ml/min. After the sample collection was completed, the PAC-EP was adjusted to neutral with a pH adjusting solution.

Four anti-CLDN18.2/anti-PD-L1 bispecific antibodies were purified via protein A, purified by SEC, concentrated, and then subjected to SEC to measure their purity; the summary table is shown in the following table:

**Table 19: SEC purity**

| | Basic physical and chemical properties of proteins | | | Purification yield | | SEC purity | Concentr ation | SEC purity |
|---|---|---|---|---|---|---|---|---|
| **Antibody** | **Molecular weight** | **Theoretic al pI** | **Ext. coefficie nt** | **293E transie nt transfe ction volume (ml)** | **Purific ation yield via protein A (mg/L)** | **SEC purity** | **Concent ration of concentr ated protein (mg/ml)** | **SEC purity** |
| **QD3691433 (HuQP19019 1/QP322)** | 175942.86 | 8.14 | 1.584 | 720 | 10.388 889 | 99% | 6.2 | 97% |
| **QD3701440 (HuQP19219 3/QP322)** | 169329.62 | 8.17 | 1.64 | 150 | 52.8 | 96.50 % | 6.2 | 96% |
| **QD3711461 (HuQP20720 8/QP322)** | 170084.37 | 8.03 | 1.521 | 600 | 64.4 | 93.30 % | 7.2 | 99% |
| | | | | | | | | |
| **QD3721116 (QP11151116/ QP322)** | 168652.48 | 8.16 | 1.735 | 600 | 51.06 | 99% | 1.5 | 100% |

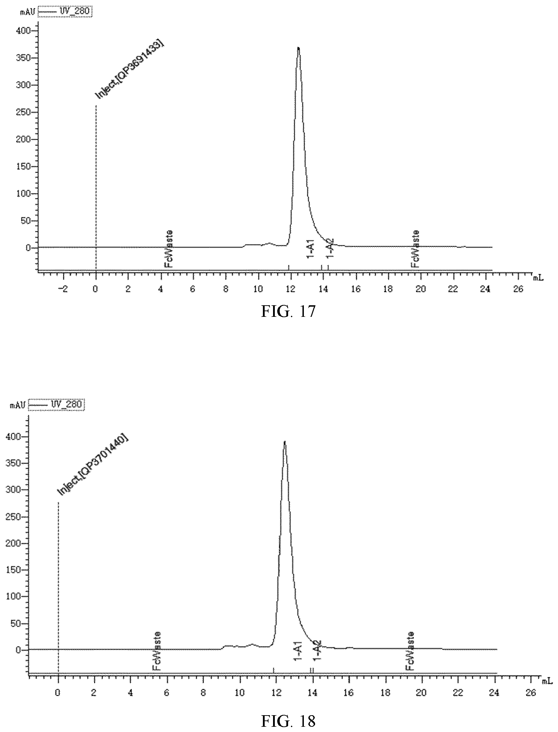

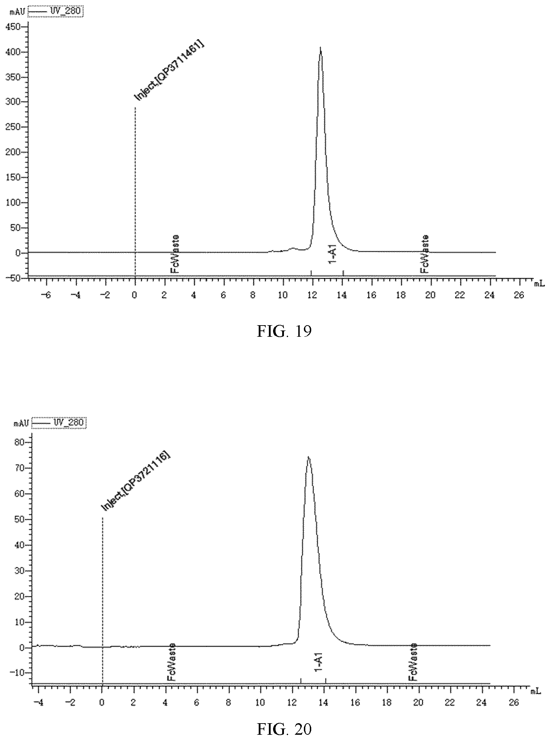

SEC purity identification was completed by HPLC, and the 4 molecular maps in the table are as follows: as shown in FIG. 17, FIG. 18, FIG. 19 and FIG. 20. QP3691433 was shown in FIG. 17, QP3701440 was purified as FIG. 18, QP3711461 was purified as FIG. 19, and QP3721116 was purified as FIG. 20. The results show that the transient expression yield of anti-Claudin 18.2/PD-L1 bispecific antibody was very good, and the SEC purity was quite good. The physical and chemical properties of the concentrated proteins are stable.

### 4. Binding activity of anti-CLDN18.2/anti-PD-L1 bispecific antibodies (PD-L1 binding)

### PD-L1 Binding ELISA:

To coat 50µl/well of antibodies QP322 0.75µg/ml, QP11801181 1.5µg/ml, QP3691433, QP3701440, QP3711461, and QP3721116 overnight at 4°C. PBS 3 times. Blocking: 3% BSA 250µl/well, placed at RT for 1 h. 1 µg/ml Biotin QP004.3(biotin-PDL1-FC) were incubated respectively with different concentrations in 1:5 dilution at RT for 1 h. PBST 6 times, PBS 3 times. Incubation of secondary antibody: HRP-strepavidin (1:5000) 50 µl/well, PBST 6 times, PBS 3 times. Color development: TMB 100µl/well, color development was allowed for 10 min. 2M H₂SO₄ 50 µl/well was added for stop reaction.

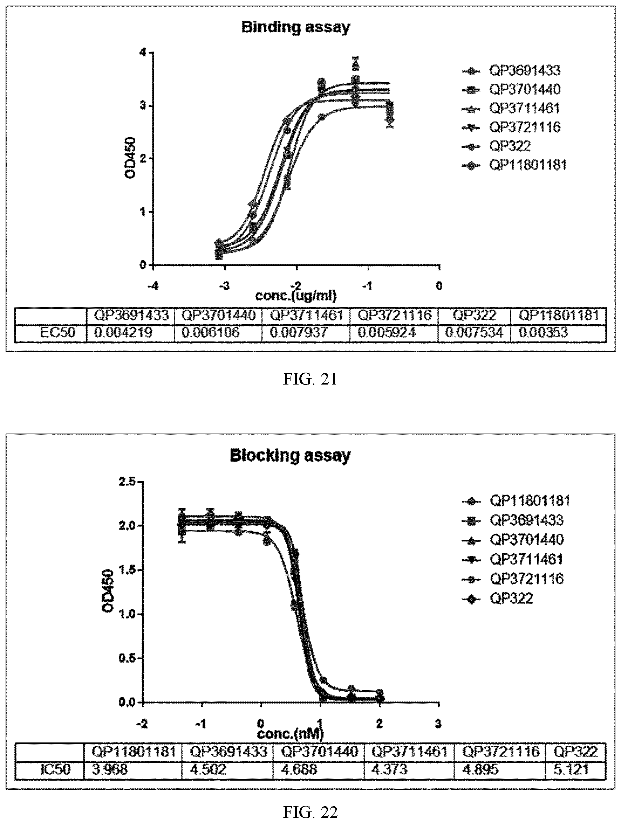

The results were shown in FIG. 21 and Table 20. ELISA results show that the PD-L1 nanobody in anti-Claudin 18.2/PD-L1 bispecific antibodies QP3691433, QP3701440, QP3711461, and QP3721116 was fused to the C-terminal of FC fragment, and the PD-L1 nanobody in isotype control (QP322) was fused to the N-terminal of FC fragment, in which both of them bind to human PD-L1 protein and their binding EC50 were equivalent.

**Table 20: PD-L1 Binding ELISA**

| conc.(µg/m l) | QP3691433 | | QP3701440 | | QP3711461 | | QP3721116 | | QP322 | | QP1180118 1 | |
|---|---|---|---|---|---|---|---|---|---|---|---|---|
| 1 | 3.255 | 3.093 | 3.411 | 3.338 | 3.300 | 3.244 | 3.191 | 3.054 | 3.138 | 3.164 | 2.960 | 3.025 |
| 0.2 | 3.009 | 2.833 | 2.970 | 2.981 | 3.059 | 3.011 | 3.033 | 2.964 | 2.908 | 2.860 | 2.598 | 2.877 |
| 0.066667 | 3.362 | 3.289 | 3.493 | 3.456 | 3.904 | 3.683 | 3.450 | 3.540 | 3.031 | 3.067 | 3.105 | 3.227 |
| 0.022222 | 3.471 | 3.450 | 3.366 | 3.305 | 3.297 | 3.214 | 3.309 | 3.358 | 2.795 | 2.783 | 3.421 | 3.445 |
| 0.007407 | 2.527 | 2.550 | 2.109 | 2.018 | 1.652 | 1.687 | 2.165 | 2.142 | 1.440 | 1.657 | 2.729 | 2.713 |
| 0.002469 | 0.976 2 | 0.927 7 | 0.695 8 | 0.646 1 | 0.4895 | 0.476 1 | 0.759 2 | 0.737 0 | 0.432 9 | 0.520 8 | 1.133 | 1.168 |
| 0.000823 | 0.299 0 | 0.288 7 | 0.222 0 | 0.222 5 | 0.1826 | 0.179 2 | 0.310 7 | 0.315 7 | 0.184 2 | 0.200 1 | 0.417 6 | 0.428 5 |
| 0 | 0.136 5 | 0.129 9 | 0.126 6 | 0.110 8 | 0.0950 0 | 0.102 2 | 0.216 1 | 0.208 3 | 0.112 4 | 0.120 7 | 0.205 8 | 0.230 4 |
| EC50 | 0.004219 | | 0.006106 | | 0.007937 | | 0.005924 | | 0.007534 | | 0.00353 | |

### 5. Blocking activity of anti-CLDN18.2/anti-PD-Ll bispecific antibodies (PD-Ll/PD-1 blocking)

### PD-L1/PD-1 blocking ELISA

Coating: antibody QP1138 2µg/ml 50µl/well, overnight at 4°C. PBS 3 times. Blocking: 3% BSA 250µl/well, placed at RT for 1 h. 2 µg/ml Biotin QP004.3(biotin-PDL1-FC) and different concentrations of QP322 15 µg/ml, QP11801181 30 µg/ml, QP3691433, QP3701440, QP3711461, and QP3721116 36µg/ml were prepared respectively, diluted at 1:3, uniformly mixed with equal volume, and placed at RT for 1 h. PBST 3 times, PBS 3 times. Incubation of secondary antibody: HRP-strepavidin (1:5000) 50 µl/well, PBST 3 times, PBS 3 times. Color development: TMB 100µl/well, color development was allowed for 10 min. 2M H₂SO₄ 50 µl/well was added for stop reaction.

The results were shown in FIG. 22 and Table 21. The ELISA results show that the PD-L1 nanobody in anti-Claudin 18.2/PD-L1 bispecific antibodies QP3691433, QP3701440, QP3711461,and QP3721116 was fused to the C-terminal of FC, and the PD-L1 nanobody in isotype control (QP322) was fused to the N-terminal of FC, in which both of them can block the binding of human PD-L1 protein to PD-1 protein and their inhibitory IC50 were equivalent.

**Table 21: ELISA blocking results of PD-L1/PD-1**

| conc.(n M) | QP11801181 | | QP3691433 | | QP3701440 | | QP3711461 | | QP3721116 | | QP322 | |
|---|---|---|---|---|---|---|---|---|---|---|---|---|
| 100.000 0 | 0.0451 0 | 0.0472 0 | 0.0366 0 | 0.0391 0 | 0.0372 0 | 0.0360 0 | 0.0337 0 | 0.0340 0 | 0.110 2 | 0.124 2 | 0.0416 0 | 0.0428 0 |
| 33.3333 | 0.0692 0 | 0.0592 0 | 0.0556 0 | 0.0634 0 | 0.0512 0 | 0.0473 0 | 0.0453 0 | 0.0468 0 | 0.158 6 | 0.158 1 | 0.0569 0 | 0.0661 0 |
| 11.1111 | 0.0942 0 | 0.0798 0 | 0.0800 0 | 0.0778 0 | 0.0612 0 | 0.0662 0 | 0.0649 0 | 0.0588 0 | 0.237 7 | 0.270 7 | 0.0947 0 | 0.1085 |
| 3.7037 | 1.060 | 1.138 | 1.501 | 1.469 | 1.511 | 1.466 | 1.354 | 1.417 | 1.486 | 1.598 | 1.630 | 1.732 |
| 1.2346 | 1.792 | 1.843 | 2.031 | 2.097 | 1.818 | 1.929 | 2.017 | 1.983 | 2.094 | 1.999 | 2.028 | 2.002 |
| 0.4115 | 1.927 | 1.933 | 2.046 | 2.046 | 2.041 | 1.999 | 2.105 | 2.123 | 2.154 | 2.081 | 2.099 | 2.077 |
| 0.1372 | 2.048 | 1.970 | 2.069 | 2.134 | 2.129 | 2.013 | 2.123 | 2.055 | 2.194 | 2.061 | 2.002 | 2.055 |
| 0.0457 | 2.062 | 1.819 | 1.992 | 1.910 | 2.194 | 2.002 | 2.021 | 2.078 | 2.152 | 2.076 | 1.931 | 2.105 |
| EC50 | 3.968 | | 4.502 | | 4.688 | | 4.373 | | 4.895 | | 5.121 | |

### 6. Blocking activity of anti-CLDN18.2/anti-PD-L1 bispecific antibodies (PD-L1/CD80 blocking)

### PD-L1/CD80 blocking ELISA

To coat CD80-FC 4µg/ml overnight at 4°C. It was followed by washing with PBS for 3 times, and blocking with 5% skim milk powder at RT for 1h. Biotin-QP004+CHO14 (10µg/ml, 1:5 dilution) having final concentration of 0.5µg/ml was incubated at RT for 1h. To wash with PBST for 5 times. HRP-Strepavidin (1:5000) was washed with PBST for 6 times and washed with PBS for 3 times. TMB for color development. Note: CHO14 protein is QP3711461 which is a protein expressed using CHO cells.

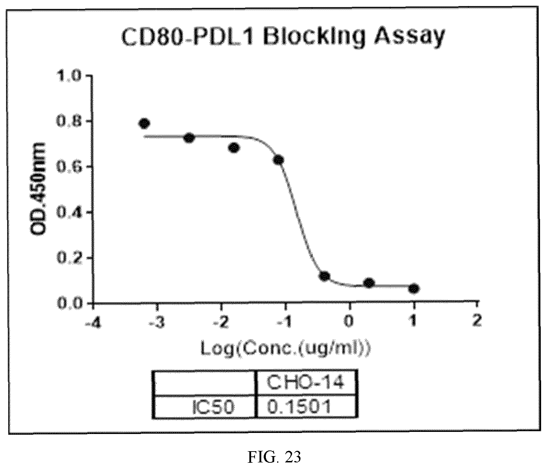

The results were shown in FIG. 23 and Table 22. The ELISA results show that the anti-Claudin 18.2/PD-L1 bispecific antibody CHO14(QP3711461) of the present disclosure can block the binding ofPD-L1 and CD80.

**Table 22: ELISA blocking results of PD-L1/CD80**

| coat CD80-FC 4µg/ml | | |
|---|---|---|
| conc.(µg/ml) | Biotin-QP004.3 | 0.5µg/ml Biotin-QP004+ CHO-14 |
| 10 | 3.1933 | 0.0577 |
| 2 | 1.8659 | 0.0856 |
| 0.4 | 0.5909 | 0.1167 |
| 0.08 | 0.1792 | 0.6281 |
| 0.016 | 0.0668 | 0.6836 |
| 0.0032 | 0.0468 | 0.7275 |
| 0.00064 | 0.0480 | 0.7918 |
| 0 | 0.0484 | 0.7059 |

### 7. Binding activity of anti-CLDN18.2/anti-PD-L1 bispecific antibodies (the activity of CLDN18.2 was detected by ELISA)

Detection reagents: skimmed milk powder (BD, 232100), PBS (Sangon, B548117-0500); HRP-anti human IgG(H+L) (jackson, 109-035-088); TMB (Luoyang Baiaotong Experimental Materials Center, C060201); Elisa plate (costa, 9018) 1×PBS buffer: 8.00 g ofNaCl, 0.20 g ofKCl, 2.9 g ofNa₂HPO₄•12H₂O, and 0.2g of KH₂PO₄ were weighed, and dissolved in 800 mL of ddH₂O, the volume was adjusted to 1L after full dissolution, the pH was adjusted to 7.4, and the mixture was sterilized at high temperature for use. Alternatively, commercial 10 ×, 20× PBS solutions were purchased and diluted to 1 ×PBS buffer for use. Blocking solution: toward PBS, 5g of milk powder was weighed; the blocking solution should be prepared immediately before use. Stop solution (1 mol/LH₂SO₄): 109 ml of 98% concentrated H₂SO₄ was slowly added dropwise into 2000 mL of ddH₂O. 100 µl/well of TMB was added for color development at 37°C for 10 min, and the plate was placed in a shaker (120 rpm);

**Experimental steps:** cells CHOS-CLD18.2-16-2 were plated into an U-shaped plate at 2E5/well, then washing once with ice PBS, and centrifuging at 1200rpm for 3 min; VCD: 1.21E6, actually 165µl/well was plated; 200µL/well of blocking solution was added after completing cleaning and incubated on ice for 1h. The blocking was completed followed by centrifuging at 1200 rpm for 3min, discarding the supernatant, incubating CHO14 sample, diluting according to 100 µg/ml in the dilution ratio of 1:2 for 12 gradients, and finally set a blank control; adding 100 µl/well according to 100 µg/mL, 33.33333333 µg/mL, 11.11111111 µg/mL, 3.703703704 µg/mL, 1.234567901 µg/mL, 0.411522634 µg/mL, 0.137174211 µg/mL, 0.045724737 µg/mL, 0.015241579 µg/mL, 0.005080526 µg/mL, 0.001694 µg/mL, 0 µg/mL, fully mixing, incubating on ice for 2 h, and washing with ice PBS for 3 times; adding enzyme-labeled antibody: incubating HRP-anti human IgG (H+L) antibody, mixing thoroughly according to 100ul/well in the dilution ratio of 1:10000, then placing on ice for 1h and washing with ice PBS for 3 times. Addition of substrate color-developing solution: substrate color-developing solution TMB was added according to the dosage of 100 µL/well, then placing in a shaker at 200 rpm, and developing color at 35°C away from light for 10 min. Stop reaction: the color development was completed followed by rapidly adding stop solution according to the dosage of 100 µL/well to stop the reaction. Detection: after centrifugation at 3500 rpm for 5 min, 120 µl of supernatant was transferred to an Elisa plate, and the OD value at A450nm was measured on a microplate reader; the results were analyzed using graphpad prism software.

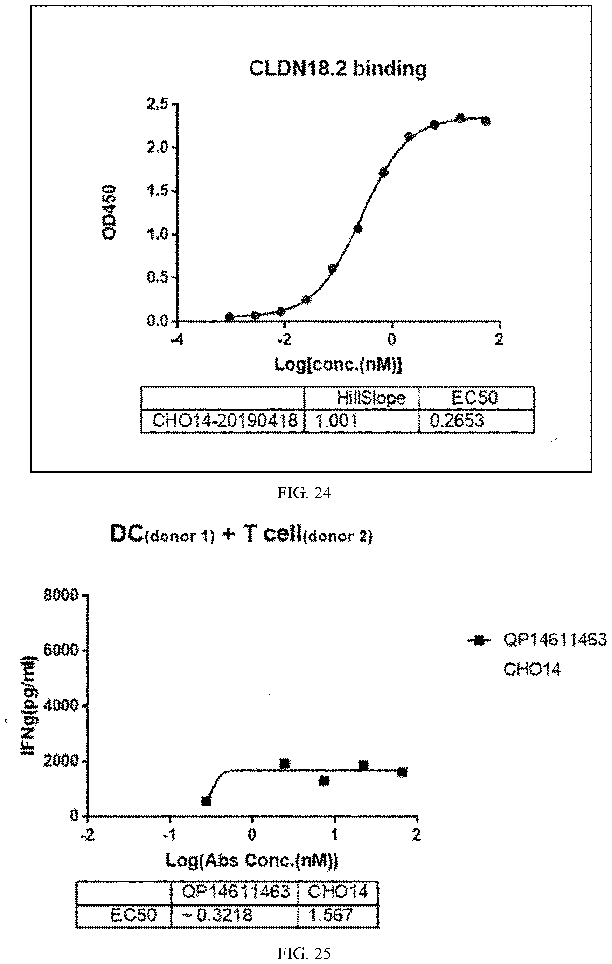

The binding EC50 of CHO14 to CHOS-CLDN18.2 high expression cell strain is 0.2653 nM. It is proved that candidate CHO14 binds Claudin18.2, and the results are shown in FIG. 24.

### 8. Identification of PD-L1 functional activity of anti-CLDN18.2/anti-PD-L1 bispecific antibodies (Mixed Lymphocyte Reaction MLR)

**Preparation of DC (donor1) cells:** PBMCs were resuscitated, monocytes were isolated with EasySep^{™}Human Monocyte Isolation Kit(Stemcell 19359), rhGM-CSF (1000U/ml) and rhIL4 (500 U/ml) were added, and the cells were cultured at 37°C for 6 days to induce iDC; the culture solution was replaced every 2-3 days and a half, and rhGM-CSF (1000 U/ml) and rhIL4 (500 U/ml) were supplemented at the same time; the collected cells were centrifuged at 300 × g for 5 min, suspended in a culture medium supplemented with rhGM-CSF (1000 U/ml) and rhIL4 (500 U/ml), and LPS (1 µg/ml) was added simultaneously; the cells were further cultured at 37°C for 1 day to induce mature DCs; and the cells were collected and counted for later use. **Preparation of T (donor 2) cells:** PBMCs were resuscitated, and CD4+ T cells were isolated using FasySep^{™}Human CD4+T Cell Isolation Kit (Stemcell 17952). **Preparation of antibody:** the antibody (initial concentration 10µg/ml) was diluted with culture medium with gradient dilution of 1:5 for 6 concentrations. The DC cells and T cells were mixed at a ratio of 1:10, different concentrations of antibodies were added, mixed and cultured, the expression of IL2 in the culture supernatant was detected on the second day, and the expression of IFNg in the culture supernatant was detected on the 5th day.

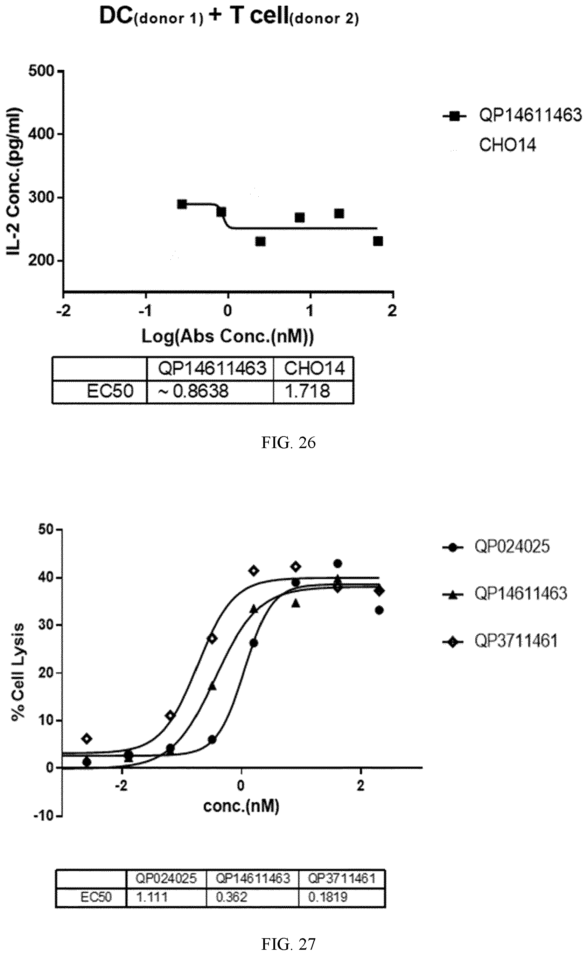

In the mixed lymphocyte reaction experiment, the candidate molecule CHO14 has obvious antibody concentration dependence on the concentrations of cytokines IFNγ and IL-2 produced after T cell activation. The biological functions of PD-L1 antibody in the candidate molecule CHO14 were demonstrated, and CHO14 can significantly promote T cell proliferation. It was shown in FIGs. 25 and 26.

### 9. PBMC-mediated Cell killing of anti-CLDN18.2/anti-PD-L1 bispecific antibodies

**Preparation of target cell (HCC827-CLDN18.2):** HCC827-CLDN18.2 was digested with trypsin at 1000 rpm for 5 min. It was followed by replacing with fresh culture medium, plating on a 96-well plate in 20000 cells/well, and incubating at 37°C in 5% CO₂ overnight. **Preparation of antibody:** antibody (200 nM-0.000512 nM, 0) were diluted with culture medium with 1:5 gradient for 10 concentrations. The culture medium in the 96-well plate was suck out, and 70 Ul/well of the above diluted antibodies of each concentration were added, and duplicate wells were set for each concentration. **Preparation of PBMC:** the PBMCs resuscitated on the first day were centrifuged, suspended in the culture medium, and counted. 70Ul/well of cells according to PBMC:target cell=50:1 were added into the above well plate, and incubated at 37°C for 4 hours. To Max lysis well, 15 µl lysis buffer (1%Triton-X100) was added, and incubated at 37 degrees for 10 min.

**Preparation of LDH reagents:** 96-well plate was centrifuged at 200 g for 5 minutes, and 100µl/well of supernatant was transferred to a new transparent 96-well plate. An LDH cytotoxicity assay kit (cayman, 10008882-480well) was taken out to prepare a Reaction Solution, 100µl/well was added, and gently shaken at 37 degrees for 30 minutes. Absorption readings at 490 nm were obtained, and the data were analyzed according to the formula Con(µg/ml)%Maximal signal=(Test-Control)/(Max-Control)-Con(0 µg/ml)%Maximal signal.

The results are as shown in FIG. 27, Table 23 and Table 24. In ADCC experiment, both anti-Claudin 18.2 monoclonal antibody QP14611463 and anti-CLDN18.2/anti-PD-L1 bispecific antibody QP3711461 showed concentration-dependent PBMC-mediated killing on human lung cancer cells HCC827-CLDN18.2 which recombinantly express Claudin 18.2 and naturally express PD-L1. The killing EC50 of bispecific antibody QP3711461 was superior to that of monoclonal antibody QP14611463 against Claudin 18.2.

**Table 23: Readings at 490nm**

| | | | | | | | | | | | | |
|---|---|---|---|---|---|---|---|---|---|---|---|---|
| MA X | 200 | 40 | 8 | 1.6 | 0.3 2 | 0.0 64 | 0.01 28 | 0.002 56 | 0.000 512 | 0 | | |
| 1.2 84 | 0.6 78 | 0.7 61 | 0.7 27 | 0.6 2 | 0.4 48 | 0.4 33 | 0.42 2 | 0.408 | 0.414 | 0.3 08 | T+E+ Ab | QP0240 25 |
| 0.3 13 | 0.3 09 | 0.3 01 | 0.2 96 | 0.2 89 | 0.2 98 | 0.3 12 | 0.32 | 0.302 | 0.31 | | T+Ab | |
| 1.0 53 | 0.7 18 | 0.7 34 | 0.6 91 | 0.6 81 | 0.5 44 | 0.4 28 | 0.41 6 | 0.413 | 0.37 | 0.2 88 | T+E+ Ab | QP1461 1463 |
| 1.1 22 | 0.3 02 | 0.2 84 | 0.2 9 | 0.3 01 | 0.2 94 | 0.3 01 | 0.30 8 | 0.319 | 0.317 | 0.2 99 | T+Ab | |
| 1.0 69 | 0.6 97 | 0.7 07 | 0.7 43 | 0.7 47 | 0.6 32 | 0.5 01 | 0.41 4 | 0.477 | 0.414 | 0.3 93 | T+E+ Ab | QP3711 461 |
| 1.2 12 | 0.7 28 | 0.7 3 | 0.7 68 | 0.7 49 | 0.6 24 | 0.4 8 | 0.42 5 | 0.422 | 0.392 | 0.4 01 | T+E+ Ab | |
| 0.0 5 | 0.3 37 | 0.3 29 | 0.3 26 | 0.3 37 | 0.2 72 | 0.3 17 | 0.31 9 | 0.335 | 0.327 | 0.3 | | |

**Table 24: Killing percentage**

| conc.(nM) | QP024025 | QP14611463 | QP3711461 |
|---|---|---|---|
| 200.00 | 33.192% | 37.917% | 37.267% |
| 40.00 | 42.996% | 39.807% | 37.976% |
| 8.00 | 38.980% | 34.727% | 42.346% |
| 1.60000 | 26.341% | 33.546% | 41.460% |
| 0.32000 | 6.024% | 17.364% | 27.286% |
| 0.06400 | 4.252% | 3.662% | 11.044% |
| 0.01280 | 2.953% | 2.244% | 2.658% |
| 0.00256 | 1.299% | 1.890% | 6.201% |
| 0.00051 | 2.008% | -3.189% | 0.709% |
| 0 | -10.513% | -12.875% | 0.000% |

### 10. In vivo efficacy study of anti-CLDN18.2/anti-PD-L1 bispecific antibodies in immune target humanized transgenic mouse C57BL/6-hPDL1 model MC38-hPDL1-mClaudin18.2.

Experimental method: colon cancer cells MC38-hPDL1(Tg)-mClaudin18.2(Tg) cells of mice in logarithmic growth phase (the cell overexpresses human PDL1 and mouse Claudin18.2, with mouse PDL1 knocked out at the same time) were taken, the culture medium was removed, the cells were washed with PBS twice, and inoculated subcutaneously in the right flank of C57BL/6-hPDL1 mice with an inoculation amount of 5×10⁵/100µL/mouse. The mice after inoculation were observed and the tumor growth was monitored; on the 8th day after inoculation, when the mean tumor volume reached 82.85 mm³, they were randomly divided into 4 groups with 9 mice in each group according to the tumor volume. The grouping day was defined as D0 day, and administration was started on D0 day.

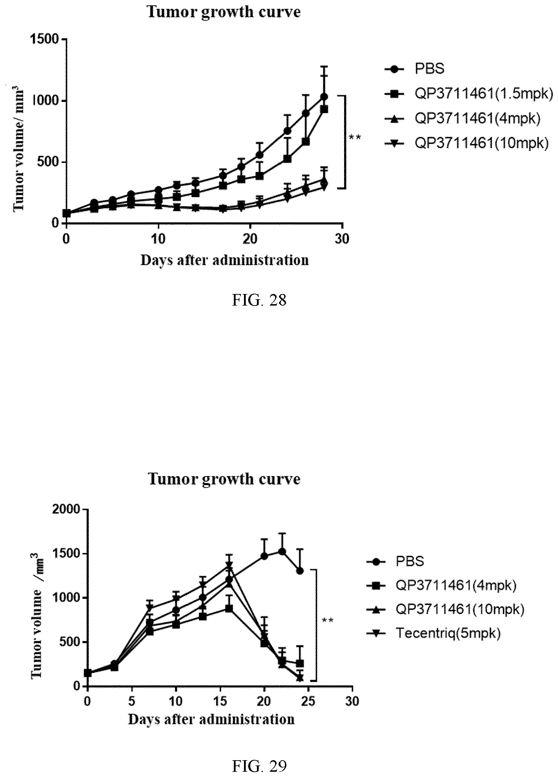

Experimental results: as shown in FIG. 28 and Table 25.

**Table 25: Tumor volume**

| Group | Dosage (mg/kg) | Tumor volume (mean±standard error, mm³) | Tumor growth inhibition rate (%) | P value |
|---|---|---|---|---|
| | | | | vs vehicle |
| Vehicle control group (PBS) | -- | 1033.97±170.88 | -- | -- |
| QP3711461 | 1.5 | 932.52±347.62 | 12.19% | 0.797 |
| QP3711461 | 4 | 360.92±97.45 | 61.81% | 0.005** |
| QP3711461 | 10 | 294.5±137.09 | 69.53% | 0.005** |

The molecule to be detected is anti-CLDN18.2/anti-PD-L1 bispecific antibody QP3711461, and the administration dosage was 1.5 mpk, 4 mpk, 10 mpk, BIW×3, administered i.v. On the 28th day after administration, the mean tumor volume of PBS group (negative control group) reached 1033.97 mm³, the mean tumor volume of QP3711461(1.5 mpk) group was 932.52 mm³ and TGI=12.19%, the mean tumor volume of QP3711461(4 mpk) group was 360.92 mm³ and TGI=61.81%; the mean tumor volume of QP3711461(10 mpk) group was 294.50 mm³and TGI=69.53%; there are statistically extremely significant differences in the mean tumor volumes of 4mpk group, 10 mpk group and PBS group (t test, p<0.01).

The experiment demonstrates that our anti-CLDN18.2/anti-PD-L1 bispecific antibody showed superior anti-tumor ability in immune target humanized transgenic mouse MC38-hPDL1-mClaudin18.2 model.

### 11. In vivo efficacy study of anti-CLDN18.2/anti-PD-L1 bispecific antibodies in immune system humanized mouse PBMC engrafted-NCG model HCC827-hClaudin18.2

Experimental method: HCC827-hClaudin 18.2 cells of stably transfected cell strain of human lung adenocarcinoma cell in logarithmic growth phase (the cells naturally highly express human PDL1 and overexpress human Claudin18.2) were taken, the culture medium was removed, the cells were washed with PBS twice, and inoculated subcutaneously in the right flank of NCG mice with an inoculation amount of 5×10⁶/100µL/mouse. The mice after inoculation were observed and the tumor growth was monitored; on the 7th day after inoculation, human PBMCs (5×10⁶/100µL/mouse) were inoculated when the mean tumor volume reached about 150mm³. They were randomly divided into 4 groups with 9 mice in each group according to the tumor volume. The grouping day was defined as D0 day, and administration was started on D0 day.

Experimental results: as shown in FIG. 29 and Table 26.

**Table 26: Tumor volume in in vivo pharmacodynamics evaluation in immune system humanized mouse PBMC engrafted-NCG model HCC827-hClaudin18.2**

| Group | Dosage (mg/kg) | Tumor volume (mean±standard error, mm³) | Tumor growth inhibition rate (%) | P value |
|---|---|---|---|---|
| | | | | vs vehicle |
| Vehicle control group (PBS) | -- | 1306.80 ± 243.99 | -- | -- |
| QP3711461 | 4 | 258.51 ± 196.56 | 80.22% | 0.005** |
| QP3711461 | 10 | 104.81 ± 78.65 | 91.98% | 0.001^{∗∗} |
| Tecentriq analog | 5 | 90.90 ± 20.83 | 93.04% | 0.001^{∗∗} |

The molecule to be detected is anti-CLDN18.2/anti-PD-L1 bispecific antibody QP3711461, and the administration dosage thereof was 4mpk, 10mpk, respectively, administered by BIW×3, i.v.; and the control antibody molecule Tecentriq, with a administration dosage of 5mpk, was administered by BIWX3, i.v. On the 24th day after administration, the mean tumor volume of PBS group (negative control group) reached 1306.8mm³, the mean tumor volume of QP1461371(4mpk) group was 258.51mm³ and TGI=80.22%, the mean tumor volume of QP1461371(10mpk) group was 104.81mm³ and TGI=91.98%; the mean tumor volume of Tecentriq(5mpk) group was 90.90mm³ and TGI=93.04%, there are statistically extremely significant differences in the mean tumor volumes of QP3711461(4mpk) group, QP3711461(10mpk) group and Tecentriq(5mpk) group versus PBS group (t test, p<0.01).

The experiment demonstrates that our anti-CLDN18.2/anti-PD-L1 bispecific antibody also showed superior anti-tumor ability in HCC827-hClaudin18.2 model of immune system humanized mouse.

### 12. In vivo synergistic efficacy studyof anti-CLDN18.2/anti-PD-L1 bispecific antibodies in C57BL/6 model MC38-hPDL1-mClaudin18.2

Experimental method: colon cancer cells MC38-hPDL1(Tg)-mClaudin18.2(Tg) cells of mice in logarithmic growth phase (the cell overexpresses human PDL1 and mouse Claudin18.2, with mouse PDL1 knocked out at the same time) were taken, the culture medium was removed, the cells were washed with PBS twice, and inoculated subcutaneously in the right flank of C57BL/6 mice with an inoculation amount of 5×10⁵100µL/mouse. The mice after inoculation were observed and the tumor growth was monitored; on the 7th day after inoculation, when the mean tumor volume reached 90mm³, they were randomly divided into 4 groups with 10 mice in each group according to the tumor volume. The grouping day was defined as D0 day, and administration was started on D0 day.

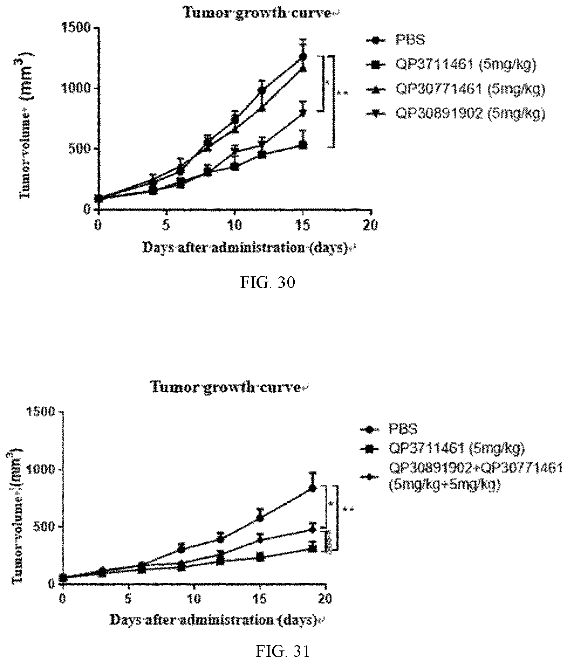

Experimental results: as shown in FIG. 30 and Table 27.

**Table 27 Tumor volume**

| Group | Remarks | Dosage (mg/kg) | Tumor volume (mean±standard error, mm³) | Tumor growth inhibition rate (%) | P value |
|---|---|---|---|---|---|
| | | | | | vs vehicle |
| Vehicle control group (PBS) | | -- | 1262.27±144.82 | -- | -- |
| QP3711461 | Bispecificity | 5 | 532.87±122.10 | 62.24% | 0.001** |
| QP30771461 | Only has the activity against CLDN18.2, but does not bind PD-L1 | 5 | 1173.62±190.39 | 7.59% | 0.715 |
| QP30891902 | Only has the activity against PD-L1, but does not bind CLDN18.2 | 5 | 794.75±99.53 | 39.63% | 0.016* |

The molecules to be detected are anti-CLDN18.2/anti-PD-L1 bispecific antibody QP3711461, the molecule QP30771461 having binding capacity for mutated PD-L1 (QP3711461-APDL1 null) and the molecule QP30891902 having binding capacity for mutated Claudin 18.2 (QP3711461-ΔClaudin 18.2 null), with an administration dosage of *5 mg*/*kg,* Q2D×6, administered *ip..* On the 13th day after administration, the mean tumor volume of PBS group (negative control group) reached 1262.27 mm³, the mean tumor volume of QP3711461 group was 532.87mm³ and TGI=62.24%, the mean tumor volume of QP30771461 group was 1173.62mm³ and TGI=7.59%; the mean tumor volume of QP30891902 group was 794.75mm³ and TGI=39.63%; there are statistically extremely significant difference in the mean tumor volumes of QP30771461 group versus PBS group (ttest, p<0.01); and there are statistically significant difference in the mean tumor volumes of mutated molecule QP30891902 group versus PBS group (t test, p<0.05).

The experiment demonstrates that the anti-CLDN18.2/anti-PD-L1 bispecific antibody had synergistic effect in MC38-hPDL1-mClaudin18.2 model of C57BL/6 mice, and showed superior antitumor activity as compared with Claudin18.2 monoclonal antibody and PD-L1 monoclonal antibody.

### 13. In vivo synergistic efficacy studyof anti-CLDN18.2/anti-PD-L1 bispecific antibodies in C57BL/6 model MC38-hPDL1-mClaudin18.2

Experimental method: colon cancer cells MC38-hPDL1(Tg)-mClaudin18.2(Tg) cells of mice in logarithmic growth phase (the cell overexpresses human PDL1 and mouse Claudin18.2, with mouse PDL1 knocked out at the same time) were taken, the culture medium was removed, the cells were washed with PBS twice, and inoculated subcutaneously in the right flank of C57BL/6 mice with an inoculation amount of 5×10⁵/100µL/mouse. The mice after inoculation were observed and the tumor growth was monitored; on the 6th day after inoculation, they were randomly divided into 3 groups with 15 mice in each group according to the tumor volume. The grouping day was defined as D0 day, and administration was started on D0 day.

Experimental results: as shown in FIG. 31 and Table 28.

**Table 28 Tumor volume**

| Group | Dosage (mg/kg) | Tumor volume (mean±standard error, mm³) | Tumor growth inhibition rate (%) | P value |
|---|---|---|---|---|
| | | | | vs vehicle |
| Vehicle control group (PBS) | -- | 838.76±133.14 | -- | -- |
| QP3711461 | 5 | 313.63±59.33 | 67.15% | 0.0019** |
| QP30771461+ QP30891902 | 5+5 | 478.61±55.42 | 45.98% | 0.0220* |

The molecules to be detected were anti-CLDN18.2/anti-PD-L1 bispecific antibody QP3711461, the molecule QP30771461 having binding capacity for mutated PD-L1 (QP3711461-ΔPDL1 null) and the molecule QP30891902 having binding capacity for mutated Claudin 18.2 (QP3711461-ΔClaudin 18.2 null), with respective administration dosages of 5 mg/kg (QP3711461 group) and 5 mg/kg+5 mg/kg (combination administration group of QP30771461+ QP30891902), Q3D×6, administered *iv*.. On the 19th day after administration, the mean tumor volume of PBS group (negative control group) reached 838.76 mm³, the mean tumor volume of QP3711461 group was 313.63 mm³ and TGI=67.15%, the mean tumor volume of combination administration group of QP30771461+QP30891902 was 478.61 mm³ and TGI=45.98%; there are statistically extremely significant difference in the mean tumor volumes of QP3711461 group versus PBS group (t test, p<0.01); and there were statistically significant difference in the mean tumor volumes of the QP30771461+QP30891902 group of combination administration of mutated molecules versus PBS group (t test, p<0.05).

The experiment demonstrated that the anti-CLDN18.2/anti-PD-L1 bispecific antibody had synergistic effect in MC38-hPDL1-mClaudin18.2 model of C57BL/6 mice, and showed superior antitumor activity as compared with the combined group of Claudin18.2 monoclonal antibody and PD-L1 monoclonal antibody (p=0.052).

All the documents referred to in the present disclosure are incorporated by reference in the present application as if each document was individually incorporated by reference. In addition, it should be understood that after reading the teachings of the present disclosure described above, a skilled person in the art can make various changes or modifications of the present disclosure, and these equivalent forms also fall into the scope as defined by the appended claims of the present application.

## Claims

1. A bispecific antibody targeting human claudin18.2 and human PD-L1 proteins, comprising:
an anti-human claudin18.2 antibody moiety and an anti-PD-L1 antibody moiety.

2. The bispecific antibody according to claim 1, wherein complementarity determining regions (CDRs) of the anti-human claudin18.2 antibody comprise:
HCDR1 as shown in SEQ ID NO: 93, 75, 79, 83 or 87,
HCDR2 as shown in SEQ ID NO: 94, 76, 80, 84 or 88, and
HCDR3 as shown in SEQ ID NO: 95, 77, 81, 85 or 89; and
LCDR1 as shown in SEQ ID NO: 90 or 72,
LCDR2 as shown in SEQ ID NO: 91 or 73, and
LCDR3 as shown in SEQ ID NO: 92, 74, 78, 82 or 86.

3. The bispecific antibody according to claim 1, wherein 3 heavy chain CDRs and 3 light chain CDRs of the anti-human claudin18.2 antibody are selected from a group consisting of:
(Z1) HCDR1, HCDR2 and HCDR3 as shown in SEQ ID Nos: 93, 94 and 95; and LCDR1, LCDR2 and LCDR3 as shown in SEQ ID Nos: 90, 91 and 92;
(Z2) HCDR1, HCDR2 and HCDR3 as shown in SEQ ID Nos: 75, 76 and 77; and LCDR1, LCDR2 and LCDR3 as shown in SEQ ID Nos: 72, 73 and 74;
(Z3) HCDR1, HCDR2 and HCDR3 as shown in SEQ ID Nos: 79, 80 and 81; and LCDR1, LCDR2 and LCDR3 as shown in SEQ ID Nos: 72, 73 and 78;
(Z4) HCDR1, HCDR2 and HCDR3 as shown in SEQ ID Nos: 83, 84 and 85; and LCDR1, LCDR2 and LCDR3 as shown in SEQ ID Nos: 72, 73 and 82;
(Z5) HCDR1, HCDR2 and HCDR3 as shown in SEQ ID Nos: 87, 88 and 89; and LCDR1, LCDR2 and LCDR3 as shown in SEQ ID Nos: 72, 73 and 86.

4. The bispecific antibody according to claim 1, wherein the anti-PD-L1 antibody is a single-domain antibody.

5. The bispecific antibody according to claim 4, wherein 3 complementarity determining regions (CDRs) of the single-domain antibody include: HCDR1 as shown in SEQ ID NO: 69, HCDR2 as shown in SEQ ID NO: 70 or 96 and HCDR3 as shown in SEQ ID NO: 71.

6. The bispecific antibody according to any one of claims 1-5, wherein the bispecific antibody is a dimer composed of two monomers having structure shown in formula I from an N terminal to a C terminal: 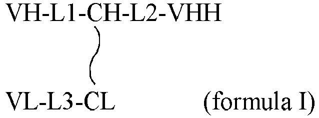 wherein,
L1, L2 and L3 are each independently a bond or a linker element;
VH represents a heavy chain variable region of the anti-human claudin18.2 antibody;
VL represents a light chain variable region of the anti-human claudin18.2 antibody;
CH represents a heavy chain constant region of the anti-human claudin18.2 antibody;
CL represents a light chain constant region of the anti-human claudin18.2 antibody;
VHH represents an anti-PD-L1 single-domain antibody;
"-"represents a peptide bond;
"∼" represents a disulfide bond or covalent bond.

7. The bispecific antibody according to any one of claims 1-6, wherein the amino acid sequence of the heavy chain variable region (VH) of the anti-human claudin18.2 antibody is as shown in SEQ ID NO: 31, SEQ ID NO: 4, SEQ ID NO: 5, SEQ ID NO: 6, SEQ ID NO: 11, SEQ ID NO: 12, SEQ ID NO: 13, SEQ ID NO: 17, SEQ ID NO: 18, SEQ ID NO: 19, SEQ ID NO: 24, SEQ ID NO: 25, SEQ ID NO: 26, SEQ ID NO: 27, SEQ ID NO: 32, SEQ ID NO: 33 or SEQ ID NO: 34.

8. The bispecific antibody according to any one of claims 1-6, wherein the amino acid sequence of the light chain variable region (VL) of the anti-human claudin18.2 antibody is as shown in SEQ ID NO: 29, SEQ ID NO: 1, SEQ ID NO: 2, SEQ ID NO: 3, SEQ ID NO: 7, SEQ ID NO: 8, SEQ ID NO: 9, SEQ ID NO: 10, SEQ ID NO: 14, SEQ ID NO: 15, SEQ ID NO: 16, SEQ ID NO: 20, SEQ ID NO: 21, SEQ ID NO: 22, SEQ ID NO: 23, SEQ ID NO: 28 or SEQ ID NO: 30.

9. The bispecific antibody according to any one of claims 4-6, wherein the amino acid sequence of the anti-PD-L1 single-domain antibody (VHH) is as shown in SEQ ID NO: 51, SEQ ID NO: 49, SEQ ID NO: 50, SEQ ID NO: 52, SEQ ID NO: 53 or SEQ ID NO: 54.

10. The bispecific antibody according to claim 6, wherein the amino acid sequence of an L chain (VL-L3-CL) of the bispecific antibody is as shown in SEQ ID NO: 63, SEQ ID NO: 59, SEQ ID NO: 61 or SEQ ID NO: 65; and the amino acid sequence of an H chain (VH-L1-CH-L2-VHH) of the bispecific antibody is as shown in SEQ ID NO: 64, SEQ ID NO: 60, SEQ ID NO: 62 or SEQ ID NO: 66.

11. The bispecific antibody according to any one of claims 1-10, wherein the bispecific antibody is a partially or fully humanized antibody.

12. The bispecific antibody targeting human claudin18.2 and human PD-L1 proteins according to claim 1, wherein:
the sequence thereof is as shown in SEQ ID NO: 59, SEQ ID NO: 60, SEQ ID NO: 61, SEQ ID NO: 62, SEQ ID NO: 63, SEQ ID NO: 64, SEQ ID NO: 65 or SEQ ID NO: 66.

13. The bispecific antibody targeting human claudin18.2 and human PDL1 proteins according to claim 1, wherein:
the anti-human claudin18.2 antibody moiety binds to an extracellular region of a human claudin18.2 protein, and the sequence of the anti-human claudin18.2 antibody moiety is as shown in: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33 or SEQ ID NO: 34.

14. The bispecific antibody targeting human claudin18.2 and human PD-L1 proteins according to claim 1, wherein the sequence of the anti-PD-L1 antibody is as shown in SEQ ID NO: 49, SEQ ID NO: 50, SEQ ID NO: 51, SEQ ID NO: 52, SEQ ID NO: 53 or SEQ ID NO: 54.

15. An isolated polynucleotide encoding the bispecific antibody according to any one of claims 1-14.

16. A vector comprising the polynucleotide according to claim 15.

17. A genetically engineered host cell, wherein the host cell contains the vector according to claim 16, or the polynucleotide according to claim 15 is integrated in the genome of the host cell.

18. A method for preparing the bispecific antibody according to any one of claims 1-14, comprising steps of:
(i) culturing the host cell according to claim 17 under suitable conditions to obtain a mixture containing the bispecific antibody; and
(ii) purifying and/or separating the mixture obtained in step (i) to obtain the bispecific antibody.

19. A pharmaceutical composition, comprising:
(a) the bispecific antibody according to any one of claims 1-14; and
(b) a pharmaceutically acceptable carrier.

20. An immunoconjugate, comprising:
(a) the bispecific antibody according to any one of claims 1-14; and
(b) a coupling moiety selected from a group consisting of a detectable label, a drug, a toxin, a cytokine, a radionuclide, or an enzyme.

21. A use of the bispecific antibody according to any one of claims 1-14 in preparing a medicament for treating cancer (or tumor), infection or immunoregulatory diseases.

22. A use of the bispecific antibody according to any one of claims 1-14 in preparing a medicament for inhibiting tumor growth.

23. The use according to any one of claim 21 or 22, wherein:
the cancer or the tumor is selected from a group consisting of colorectal cancer, breast cancer, ovarian cancer, pancreatic cancer, gastric cancer, esophageal cancer, prostate cancer, renal cancer, cervical cancer, bone marrow cancer, lymphoma, leukemia, thyroid cancer, endometrial cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, and myelodysplastic syndrome.
